# EUROPEAN PATENT APPLICATION

(11) **EP 1 469 067 A1**
(43) Date of publication of application: **20.10.2004**
(21) Application number: 03703050.9
(22) Date of filing: 24.01.2003
(51) Int. Cl.: C12N 15/09, C12Q 1/68, G01N 33/50, G01N 33/53, G01N 33/566, G01N 33/58

(54) **METHOD AND APPARATUS FOR DETECTING NUCLEIC ACID DATA**

(30) Priority: 25.01.2002 JP 2002017272; 27.08.2002 JP 2002247023
(71) Applicant: Olympus Corporation, Tokyo 151-0072 (JP)
(72) Inventor: KOIKE, Hisashi, c/o Olympus Corporation, Tokyo 151-0072 (JP); NAGAOKA, Tomonori, c/o Olympus Corporation, Tokyo 151-0072 (JP); SATOH, Takatomo, c/o Olympus Corporation, Tokyo 151-0072 (JP); KANEKO, Yoshioki, c/o Olympus Corporation, Tokyo 151-0072 (JP); HATANAKA, Midori, c/o Olympus Corporation, Tokyo 151-0072 (JP); FUKUOKA, Morinao, c/o Olympus Corporation, Tokyo 151-0072 (JP); SAKAMOTO, Hiroko, c/o Olympus Corporation, Tokyo 151-0072 (JP); YONEKAWA, Hiroyuki, c/o Olympus Corporation, Tokyo 151-0072 (JP)
(74) Representative: Wibbelmann, Jobst, Dr., Dipl.-Chem.
(86) International application number: PCT/JP2003/000668
(87) International publication number: WO 2003/062418

(57) **Abstract**

That invention provides a nucleic acid information detection method which, in a method wherein a target nucleic acid, and probes having a complementary sequence with at least a portion of the target nucleic acid sequence, are contacted with each other in order to form hybrids between the target nucleic acid and the probes, and the amount of signal generated depending on the amount of hybrids is measured in order to detect the information on the target nucleic acid, includes kinetically obtaining data of the signal.

Furthermore the invention provides a nucleic acid information detection method which, in a method wherein a perfect matched probe having a perfect complementary sequence with respect to at least part of a target nucleic acid sequence, and one or more types of imperfectly matched probes having at least one part of the perfect matched probe mutated, are contacted with the target nucleic acid in order to hybridize between the target nucleic acid and the perfect matched probe, or the imperfect matched probes, so that the information on the target nucleic acid can be detected based on the difference in binding strength of the hybrids, includes kinetically obtaining data of the signal while changing continuously or stepwise the condition for measuring or detecting the signal from the hybrids.

## Description

### TECHNICAL FIELD

The present invention relates to a nucleic acid information detection method and apparatus for kinetically detecting nucleic acid information.

### BACKGROUND ART

In recent years, gene diagnosis techniques for analyzing information on gene mutation, predicting disease, diagnosing disease, typing viruses, or the like, have greatly progressed. For example, such method has been carried out wherein DNA probes which are respectively complementary to a nucleic acid having a normal gene sequence, or a nucleic acid including an already-known mutation in the gene sequence are previously prepared, then the respective types of probes are separately hybridized with a sample DNA, for which the presence/absence of a mutation is not known, in order to determine if a probe having a larger hybridization amount has higher complementarity with the sample DNA. That is, if the hybridization amount with a probe complementary with the normal DNA is larger, the sample DNA can be assumed to be normal. On the other hand, if the hybridization amount with a probe complementary with the mutant DNA is larger, the sample DNA can be assumed to be mutated.

For detecting hybridization, for example such method has been used wherein a sample DNA is labeled with a detectable marker in order to indirectly detect the DNA amount by detecting the marker. Generally, fluorescence, radioactive isotope (RI), chemoluminescence, or the like have been used for such marker.

A further advanced gene detection system based on the hybridization reaction using such DNA complementary, has started prevailing. It is called DNA microarray. In the system, hundreds to ten hundreds of DNA probes are arranged in the array on which solutions containing nucleic acid samples are hybridized in order to detect the information on a plurality of genes all together.

Using the DNA microarray, the information on a plurality of genes can be analyzed at the same time. It has been normally used for analyzing mRNA expressions in many cases, however, on the other hand some examples include a case where it has been also applied for analyzing gene mutation or single nucleotide polymorphisms (SNPs).

It is generally quite difficult to detect single base mutations sensitively at high speed. Therefore, for example, in Japanese Unexamined Patent Application, First Publication No. 2001-50931, a method has been proposed wherein an electrochemical reaction is jointly used in the DNA microarray in order to detect single base mutation sensitively at high speed. In this reference, electrodes are respectively applied to individual array elements on the DNA microarray to which the voltages are applied so that the reactions can be performed at high speed.

Moreover, as a method for carrying out the hybridization sensitively at high speed, examples include a method disclosed in Published Japanese translation No. 2000-515251 of PCT International Publication. In this method, a metallic oxide having a porous structure is used as a reaction carrier and a solution is driven from one surface to another surface in order to increase the diffusion velocity of the sample solution so that the reactions can be performed at high speed.

However, in the conventional methods, the hybridization reaction has been performed extremely slowly (normally from several hours to many hours), so that tips having complex construction have been required for high speed detection. Accordingly, apparatus or tips for detecting nucleic acid have been made in large sizes, resulting in high cost. Moreover, the reaction temperature must be increased in order to increase the specificity, which causes a delay in reaction time and a longer detection time for the hybrid. Such problems become remarkable particularly in the case where the presence/absence of single base mutation is to be accurately detected.

Specific examples for detecting single base mutation include a method disclosed in Published Japanese translation No. 2000-511434 of PCT International Publication. In this method, a probe corresponding to a base mutation is prepared to identify the mutation using the specificity. In order to reliably detect the difference of the single base mutation, probes including mismatched nucleotide are used. In this method a phenomenon is utilized such that a Tm value difference between the perfect matched hybrid and the hybrid with a probe having a single base mutation is larger than the Tm difference between the hybrid with the probe having a single base mutation and a hybrid with a probe having a double base mutation. In the method, however, since the absolute hybridization intensity is decreased, the reaction requires longer time and there have also been problems from the point of sensitivity.

Similarly to the case of detecting single base mutations, in the case where only a single base is mutated with respect to the whole length of the DNA, there is not so much difference in the degree of complementarity between probes and a target DNA, and hence there is no substantial difference in the degree of hybridization, making impossible to detect the mutation. That is, in order to detect single base mutations, preferably the base length is shorter so as to increase the proportion of the mutation with respect to the base length. On the other hand, however, in the case where a probe comprises a too short sequence, the reaction temperature must be decreased, resulting in an increase in the amount of non-specific hybridization with the target DNA. Therefore, the problem has been such that the highly accurate detection can not be performed. In this way, in conventional methods, it has been impossible to detect a single base mutation accurately as high speed.

Moreover, in a system for detecting a single base mutation accurately at high speed, due to the directionality for limiting the reaction conditions within suitable conditions for the sequence being detected (systemic optimization), in the case of detecting many types of target sequences, the measurement must be repeated for the number of the target sequences, causing a long experimental time as a whole. Accordingly, even if the detection time for each sequence is shortened, in the case where the experimental system is to detect a plurality of sequences, the total time taken becomes long.

Therefore, it is desirable to provide a detection method wherein mutations of a plurality of types of sequences in a target nucleic acid can be detected accurately at high speed even for a single base mutation, and a method wherein more types of mutations can be detected at one time.

### DISCLOSURE OF INVENTION

The present inventors have considered and earnestly studied the various problems in the conventional techniques. Consequently, they have found that, by kinetically obtaining signal data, it becomes possible to obtain lots of more accurate and more reliable information on nucleic acid.

That is, the nucleic acid information detection method of the present invention is characterized in that, in a method wherein a target nucleic acid, and probes having a complementary sequence with at least a portion of the target nucleic acid sequence are contacted with each other in order to form hybrids between the target nucleic acid and the probes, and the amount of signal generated depending on the amount of hybrids is measured in order to detect the information on the target nucleic acid, the method includes kinetically obtaining data of the signal.

In the nucleic acid information detection method of the present invention, preferably obtaining the data of the signal is performed while changing a measurement condition or a detection condition of a reaction. More specifically while changing at least one of; a reaction temperature, a composition, a volume, and a type of reaction solution, particularly the reaction temperature.

The nucleic acid information detection method of the present invention is characterized in that in a method wherein a perfect matched probe having a perfect complementary sequence with respect to at least part of a target nucleic acid sequence, and one or more types of imperfectly matched probes having at least one part of the perfect matched probe mutated are contacted with the target nucleic acid in order to form hybrids between the target nucleic acid, and the perfect matched probe or the imperfect matched probes, so that the information on the target nucleic acid can be detected based on the difference in binding strength of the hybrids, said method includes kinetically obtaining data of the signal while changing continuously or stepwise the condition for measuring or detecting the signal from the hybrids.

In the nucleic acid information detection method, it is preferable to obtain the data of the signal while changing at least one of a reaction temperature, a composition, a volume, and a type of reaction solution, particularly the reaction temperature.

The change of the reaction temperature in the nucleic acid information detection method is preferably to increase the temperature from a temperature lower than a Tm value of the hybrids to be detected to a temperature higher than the Tm value, or a temperature cycle of one or more times comprising increase and decrease between such temperatures.

If the change of the reaction condition is to increase the temperature, the maximum value of the signal intensity and/or the amount of change in the signal intensity may be measured during the period.

The nucleic acid information detection method of the present invention may involve, in any of the aspects described above, continuously or stepwise increasing the temperature at which a signal from the hybrid is measured, measuring the change in the signal intensity from the hybrid between respective temperatures, and maintaining the temperature when the amount of change starts to decrease.

The nucleic acid information detection method of the present invention is further characterized in that, in any of the aspects described above, in an identical system where identical reaction conditions are applicable, a plurality of types of probes are used in order to detect the information on a plurality of types of nucleic acids at the same time. More specifically, DNA microarray may be used.

Furthermore, the nucleic acid sequence mutation detection method of the present invention is characterized in that said probes are a plurality of types of probes having a plurality of types of sequences and the probes have mutually overlapped sequences.

The nucleic acid sequence mutation detection method of the present invention is characterized in that, in the above nucleic acid sequence mutation detection method, the plurality of types of probes comprise overlapping probes of; a perfect matched probe having a perfect complementary sequence at least partially with the target nucleic acid sequence, one or more types of imperfect matched probes having at least one partial mutation in the perfect matched probe, and the perfect matched probe and the imperfect matched probe having an extended or shortened base sequence on both ends or one end.

The nucleic acid sequence mutation detection method of the present invention is characterized in that, in the above nucleic acid sequence mutation detection method, it further comprises a step of comparing an analysis result of a probe group having a lower Tm value among the overlapping probes with an analysis result of a probe group having a higher Tm value, thereby deciding the nucleic acid information.

For the probes having the above sequences, probes (SEQ ID NO: 56 to 69) comprising 20mer base sequences for analyzing K-ras codon12 may be used.

For the probes having the above sequences, probes (SEQ ID NO: 70 to 83) comprising 17mer base sequences for analyzing K-ras codon12 may be used.

For the probes having the above sequences, probes (SEQ ID NO: 56 to 69) comprising 17mer base sequences for analyzing K-ras codon12 and probes (SEQ ID NO: 70 to 83) comprising 20mer base sequences for analyzing K-ras codon12 may be used.

The nucleic acid information detection method of the present invention is characterized in that, in any of the aspects described above, said hybrid formation is performed by making a liquid sample including a target nucleic acid contact with a probe fixed onto a porous body.

In the nucleic acid information detection method, it is preferable to include a process for making the liquid sample reciprocate once or a plurality of times in the porous body.

In the nucleic acid information detection method of the present invention, the signal may be detected based on detection of a fluorescent marker.

In the nucleic acid information detection method of the present invention, the target nucleic acid may be any one of an oncogene, an intracellular drug resistance gene, a cell cycle regulator gene, and an apoptosis related gene, or a combination of these.

A nucleic acid information detection apparatus of present invention is characterized in comprising: a sample storage container for containing a sample including a target nucleic acid; a nucleic acid reaction carrier including a porous structure which can fix the nucleic acid and connected to the container; a driving device for mobilizing the sample under control, between the container and the nucleic acid reaction carrier without leaking; a temperature control device for controlling a reaction temperature on the reaction carrier; and a device for detecting a signal from a hybrid between a target nucleic acid and probes formed on the porous structure.

In the nucleic acid information detection apparatus of the present invention, the apparatus may further comprise: one or more solution storage containers for storing solutions connected to said nucleic acid reaction carrier and to contain types of solutions different to the sample solution including the target nucleic acid; and a device which appropriately mixes the various solutions contained in the solution storage containers and sends these to said nucleic acid reaction carrier.

In the nucleic acid information detection apparatus of the present invention, the target nucleic acid may be any one of an oncogene, an intracellular drug resistance gene, a cell cycle regulator gene, and a apoptosis related gene, or a combination of these.

### BRIEF DESCRIPTION OF THE DRAWINGS

FIG. 1 is a schematic diagram showing a nucleic acid information analyzer of the present invention.
FIG. 2 is a graph showing results of kinetic measurement of hybridization between a target nucleic acid and a perfect matched probe or imperfect matched probes, while changing the hybridization temperature. In the graph, the temperature of the signal measurement was set at 25°C in area (A), 40°C in area (B), and 60°C in area (C). In the respective temperatures, respective sample solutions were made to reciprocate towards a nucleic acid reaction carrier five times.
FIG. 3 is a graph showing results of detection of a cell line derived p53 gene according to the present invention.
FIG. 4 shows an arrangement of probes on a DNA microarray used in Example 3.
FIG. 5 shows experimental results of Example 3 for detecting K-RAS gene mutation. In the figure, the panels show the results of fluorometry, sequentially from the bottom, for 1min, 10min, 20min, 30min, and 40min after hybridization.
FIG. 6 shows an arrangement of probes on a DNA microarray used in Example 4.
FIG. 7 shows experimental results of Example 4 for detecting p53 gene and K-RAS gene at the same time. In the figure, the panels show the results of fluorometry, sequentially from the bottom, for 1min, 10min, 20min, 30min, and 40min after hybridization.
FIG. 8 shows the difference in signal from hybrids at a temperature (A'), in a system using probes A to D. In this figure, the fluorescent intensities of nucleic acid B, C and D were the highest, the fluorescent intensity of the perfect match between nucleic acid A and a probe A was the second highest, and the fluorescent intensity of a single base mismatch between nucleic acid A and the probe A was lower.
FIG. 9 shows the difference in signal from hybrids at a temperature (B'), in the system using probes A to D. The representation of the fluorescent intensity was applied correspondingly to FIG. 8. Spots without hatching denote the lowest fluorescent intensity.
FIG. 10 shows the difference in signal from hybrids at a temperature (C'), in the system using probes A to D. The representation of the fluorescent intensity was applied correspondingly to FIG. 8. Spots without hatching denote the lowest fluorescent intensity.
FIG. 11 shows the difference in signal from the hybrids at a temperature (D'), in the system using probes A to D. The representation of the fluorescent intensity was applied correspondingly to FIG. 8. Spots without hatching denote the lowest fluorescent intensity.
FIG.12 shows the profile of the temperature change when kinetically obtaining the data.
FIG. 13 shows an arrangement of probes on a DNA microarray used in Example 6.
FIG. 14 shows a comparison of the signal intensities on respective spots after the hybridization at room temperature (25°C) in Example 6.
FIG. 15 shows a comparison of the signal intensities on respective spots after the hybridization at 55°C in Example 6.
FIG. 16 shows a comparison of the signal intensities on respective spots after the hybridization at 72°C in Example 6.

### BEST MODE FOR CARRYING OUT THE INVENTION

### (Definitions)

In the present description, the following words are used in the meanings defined hereunder.

A "nucleic acid" means any one of DNA, RNA, DNA including artificial nucleotide, or RNA including artificial nucleotide.

A 'probe" means a nucleic acid fragment for examining a nucleic acid in a specimen using the hybridization reaction based on the complementarity of nucleic acid.

"A plurality types of probes" mean, in the case where there is one target gene, probes having a base sequence partially replaced or inserted with other base sequences, or defected in base sequences, and a plurality of probes using different base portions as trapped portions of the gene. In the case where there are a plurality of target genes, it means a plurality of probes of complementary nucleic acid sequences with respect to the respective genes.

A "hybrid" means a double strand formed between any one of the abovementioned nucleic acid, within the same type, or across different types, including DNA-DNA, DNA-RNA, RNA-RNA or the like.

"Information on nucleic acid" or "nucleic acid information" includes a nucleic acid sequence itself, the presence/absence of mutation in the nucleic acid sequence, a physical property which varies depending on the nucleic acid sequence (for example, Tm), and the amount of the nucleic acid (for example, number of mRNA copies).

"Signal" is a signal suitably detectable and measurable by appropriate means, including fluorescence, radioactivity, chemiluminescence, and the like.

"Kinetically perform" means not only to obtain data at a determined time point, but also to measure continuously or at respective intermittent time points.

Hereunder is a description of the structure, the implementation method, and the effects according to the embodiments of the present invention.

In a first embodiment, the nucleic acid information detection method of the present invention is characterized in that in a method wherein a target nucleic acid, and probes having a complementary sequence with at least a portion of the target nucleic acid sequence are contacted with each other in order to form hybrids between the target nucleic acid and the probes, and the amount of signal generated depending on the amount of hybrids is measured in order to detect the information on the target nucleic acid, the method includes kinetically obtaining the data of the signal.

The target nucleic acid may vary from DNA from a genome, mRNA extracted from cells, DNA amplified by PCR, plasmid DNA, and the like. It is not specifically limited unless it contains impurities which negatively affect the hybridization with the probes. However, in order to conduct a highly accurate experiment, it is preferable to use a sample highly purified by various already-known refining techniques in the technical field. Moreover, it is preferable that the sequence of the target nucleic acid being hybridized has been already known at least partially so as to compose the probes. The target nucleic acid can be labeled with a marker by mRNA reverse transcription or PCR.

The target nucleic acid and the probes can be contacted with each other by contacting solutions containing the target nucleic acid with probes immobilized on a carrier. The carrier can be one which immobilizes the nucleic acid so as not to inhibit a double strand formation with other nucleic acids.

Hybrids between the target nucleic acid and the probes may be formed under a condition determined based on the difference in binding strength depending on the sequence information of the portion being hybridized. However, for example, in the case where the condition being changed for measuring the signal is to change the temperature, the hybrids may be formed at the temperature determined based on the difference in Tm. At a temperature lower than Tm but close to Tm, although a specific double strand between the target nucleic acid and the probe is easily formed and non-specific double strand is not easily formed, it takes time for the hybrid formation. On the other hand, at a temperature further lower than the temperature at which such a specific double strand is easily formed, the reaction time for the double strand formation is shortened, however, the amount of the non-specific binding between the target nucleic acid and probes is increased. Therefore, the temperature setting for the hybridization reaction is determined in the balance between the required experimental accuracy and the required reaction time. Moreover, other reaction conditions are determined similarly considering the above.

The amount of the signal generated depending on the amount of hybrid is normally measured by using a marker previously labeled into a nucleic acid being the sample. However, for example, a sample nucleic acid labeled with fluorescence, a sample nucleic acid composed from dNTP containing a radioisotope, or the like may be used. Various nucleic acid labeling techniques (either already-known or newly developed) may be used for labeling. Furthermore, it is also possible to add a reagent which binds to the hybrid double strands, after the hybrid formation, and then detect the reagent in order to detect the hybrid.

Examples for detecting hybrids by fluorescence include, a method for using primers previously labeled with fluorescent marker when composing a sample nucleic acid by PCR, and a method for using chemical reaction or enzyme to label a sample nucleic acid with fluorescent markers. A generally-used labeling method may be used for labeling with fluorescent markers.

The signal data is not obtained at a fixed time point but is obtained kinetically. More specifically, the time for kinetically obtaining the data differs depending on the reaction conditions of the hybridization reaction. However, generally it ranges from several minutes to several hours in total after starting the hybridization reaction. It is of course also possible to obtain data for total times outside of this range if the reaction conditions suit.

As described above, the embodiment of the present invention is characterized in that the data is kinetically obtained, differing from the conventional method for statically obtaining data based on the fixed time point observation. Therefore, in the present invention, it becomes possible to chase the temporal change in the hybrid formation between a target nucleic acid and the probes. By chasing this temporal change, it becomes possible to obtain lots of more accurate and more consistent information on the target nucleic acid. That is, if the data is kinetically obtained, it becomes possible to monitor the process of the reacting state, and it becomes possible to precisely detect the progress of the reaction. Then, it becomes possible to obtain more accurate nucleic acid information from the whole process of the reaction.

In the above embodiment, it is also possible to obtain the data of the signal while changing the measurement condition or the detection condition of the reaction. More specifically while changing at least one of; the reaction temperature, the composition, the volume, and the type of reaction solution, more preferably the reaction temperature. For example, if the signal data is obtained while linking with the change of the reaction temperature, it becomes possible to obtain additional information such as the amount of reaction change of the hybridization reaction, the starting of the reaction, and the response state in the reaction system with respect to the reaction condition, which is more beneficial.

Here, "change the reaction temperature" means to change the temperature in a system for hybridizing between a target nucleic acid and probes. "Change the composition of the reaction solution" means to change the composition or pH of salt, additives, or the like in the reaction solution. "Change the volume of the reaction solution" means to change the volume by adding or taking out the reaction solution from the reaction system. "Change the type of reaction solution" means to change the type of solution such as aqueous solution, alcohol solution, or the like.

The hybrid formation between a target nucleic acid and the probes is performed under optimum conditions assumed based on the sequence information. Consequently, usually it is not known whether the assumed conditions are really the optimum condition. Therefore, in a conventional nucleic acid information detection method for measuring statically, if the data is obtained under non optimum conditions, there have been problems of the reliability of the data being insufficient, and irregularities in the data, and so on.

However, similarly to the embodiment of the present invention, by changing the reaction conditions linking with kinetically obtaining the data, it becomes possible to progress the hybridization reaction under a plurality of reaction conditions and measure this with the passage of time. Therefore, in this embodiment, the conventional problems of data reliability described above are solved.

In a case where hybridization formation is progressed under a certain condition, the hybridization reaction may be monitored by kinetically obtaining data. Accordingly, it becomes possible to detect the progress of the hybridization reaction more precisely. Therefore, in the present invention wherein signal data is kinetically obtained in this manner, it becomes possible to measure the rate of the reaction change at the initial stage of the reaction so as to predict the time point when the reaction terminates. This serves to shorten the reaction time for detecting nucleic acids. Furthermore, if it is expansively utilized, it is also possible to determine the optimum reaction conditions in hybridization formation by kinetically obtaining data so as to shift the reaction condition to be more optimum, or to make use of chasing the hybridization reaction in a broad range of reaction conditions.

Examples of the embodiment of the present invention include ones to compare the transcription amount or the number of copies of a specific gene in a sample, to detect a specific sequence (normal type or mutant type) in a nucleic acid sequence, to detect a gene polymorphism represented by SNPs, to type virus or bacteria, and the like.

In another embodiment, the nucleic acid information detection method of the present invention is characterized in that,
in a method wherein a perfect matched probe having a perfect complementary sequence with respect to at least part of the target nucleic acid sequence, and one or more types of imperfectly matched probes having at least one part of the perfect matched probe mutated, are contacted with the target nucleic acid in order to hybridize between the target nucleic acid and the perfect matched probe, or the imperfect matched probes, so that the information on the target nucleic acid can be detected based on the difference in the binding strength of the hybrids, the method includes kinetically obtaining the data of the signal while changing continuously or stepwise the condition for measuring the signal from hybrids.

In this embodiment, it becomes possible to extremely accurately detect the progress of the hybridization reaction. For example, if the signal data is obtained while changing the reaction temperature continuously or stepwise, it also becomes possible to detect a single base mutation in a nucleic acid sequence only having a difference in one basepoint.

More specifically, for example, a target nucleic acid such as a PCR product is prepared, and then a perfect matched probe having a perfect complementary sequence with at least part of the natural sequence, and three different probes having different single base mutations at a same base point, are prepared. These four types of probes have respectively any one of A, T, G, C nucleotides at the same base point, and with other parts perfectly identical. Using these four types of probes, the hybridization with the target nucleic acid is measured while changing the reaction temperature. At a temperature near Tm of a hybrid of the target nucleic acid and the perfect matched probe, the rest of the hybrids of the target nucleic acid and the imperfect matched probes are unstable. Therefore, it is considered that the equilibrium is inclined to a state where the target nucleic acid and the probe are released. Consequently, at a temperature near Tm, the hybrid between the target nucleic acid and the perfect matched probe should generate the strongest signal. On the other hand, even if the hybrids between the target nucleic acid and the imperfect matched probes are present, the amount is extremely low, and hence the signal intensity is low. From such difference in the signal intensity, or presence/absence of the signal, it becomes possible to identify which base A, T, C, and G should be in the base point suspected to be mutated in the target nucleic acid, based on the signal intensity. In a conventional method, since the reaction temperature of hybridization is a fixed point, in the case where the reaction temperature is not optimum for the hybridization, it has been also considered that the imperfect matched probes might generate rather stronger signals. However, in the embodiment of the present invention, since the reaction conditions such as reaction temperature may be flexibly changed, it becomes possible to effectively avoid such false result.

In a conventional method, in order to detect and discriminate a target nucleic acid mutated only at a single base point in this manner, the reaction temperature must be increased to a temperature near Tm. Therefore a long reaction time has been required. However, in the method of the embodiment of the present invention, since the temperature can be controlled more flexibly, for example, the hybrid formation may be started at a temperature lower than Tm, and after obtaining enough hybrid amount, the reaction temperature may be increased continuously or stepwise. As a result, the hybrid reaction time can be shortened. Furthermore, by delicately controlling the temperature within a temperature range near Tm to exclude non specific hybrid formation, it becomes possible to measure more accurately.

In this embodiment of the present invention, if probes including mutations at a plurality of base points are appropriately prepared and used, it becomes possible to detect not only a single base mutation but also a plurality of base mutations at the same time.

In this manner, the abovementioned effects may be obtained by changing the reaction temperature linking with obtaining the signal data. However, similar effects may be obtained by changing, for example, the composition, the volume, or the type of reaction solution. More specifically, it is also possible to change the conditions of the hybridization reaction by changing the type of salt or the concentration of reaction solution in the hybridization reaction, or through pH gradient formation by using different buffer solutions. Such changes of the reaction solution composition affect the hybrid formation as well as the change of reaction temperature.

In the embodiment of the present invention, from the viewpoint of shortening the reaction time, the change of reaction temperature is preferably to increase the temperature. If the temperature is increased, particularly in the case where the temperature is increased starting from a temperature lower than the assumed Tm of the hybrid, even though non specific bindings are generated, the hybrid formation between a target nucleic acid and the probes is promoted. Moreover the non specific bindings may be eliminated by increasing the subsequent temperature, and hence it is considered to be more preferable. Therefore, if the temperature is increased, it becomes possible to obtain the effects such that not only the reaction time is shortened, but also the accuracy of the hybrid reaction is increased.

The change of the reaction temperature is preferably to increase the temperature from between a temperature lower than the Tm value to a temperature higher than Tm value. However, this may be further developed by making one or more temperature cycles comprising a temperature increase and a temperature decrease, from between a temperature lower than the Tm value to a temperature higher than Tm value, to thereby obtain a profile of the hybridization reaction. Accordingly it becomes possible to obtain further reliable data.

If increasing the reaction temperature, by measuring the maximum value of the increasing signal intensity, it becomes possible to qualitatively or quantitatively determine the Tm value of the hybrid. If increasing the temperature stepwise, an approximate Tm value may be determined. Therefore, if increasing the temperature in smaller steps (for example, at intervals of 1°C or less) or continuously, a more accurate Tm value may be determined. The difference in the Tm value reflects the presence/absence or the difference of mutants in the respective nucleic acid sequences so that it becomes possible to detect mutants more precisely according to the embodiment of the present invention.

Moreover, in this embodiment, if not only detecting the signal from hybrids but also measuring the amount of signal change, it becomes possible to further increase the accuracy in determination.

In another preferred embodiment, the nucleic acid information detection method of the present invention is characterized in comprising the steps of continuously or stepwise increasing the temperature at which a signal from the hybrid is measured, measuring the change of the signal intensity from the hybrid, and maintaining the temperature when the amount of change starts to decrease.

According to this embodiment, it becomes possible to detect an unknown mutation more accurately. That is, if the change in the signal intensity is detected while the reaction temperature is being changed continuously or stepwise, so as to maintain the temperature when the amount of change in the signal intensity starts to decrease, it can be considered that, while the signal intensity from the hybrid having the higher Tm value is maintained or further increased, on the other hand the signal intensity from the hybrid having the same or lower Tm value is gradually decreased. Therefore, by using the difference in the Tm value, it becomes possible to more accurately discriminate the perfect matched hybrid and the imperfect matched hybrid, so that an unknown mutation may be identified based on the sequence of the perfect matched probe.

In another embodiment, the nucleic acid information detection method of the present invention is characterized in that, in an identical system where identical reaction conditions are applicable, a plurality of types of probes are used in order to detect the information on a plurality of types of nucleic acids at the same time. More specifically, DNA microarray is used.

At this time, hybrids between the target nucleic acid and the probes may be formed near the condition depending on the sequence of the portion being hybridized in the target nucleic acid. However, for example, in the case where the condition being changed for measuring the signal is to change the temperature, the hybrids may be formed kinetically in a temperature range determined based on the Tm of the sequence on the portion being hybridized in the target nucleic acid. That is, the signal may be kinetically measured in a temperature range from a temperature lower than the lowest Tm value among a plurality of types of probes, to a temperature higher than the highest Tm value among a plurality of types of probes. Hereunder is a description of the change in the hybridization state between the target nucleic acid and of probes when kinetically changing the temperature, with reference to drawings.

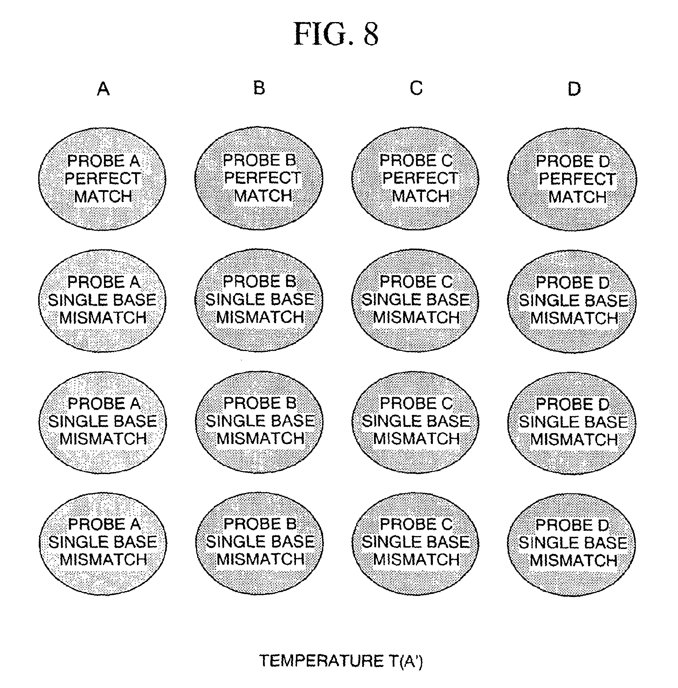

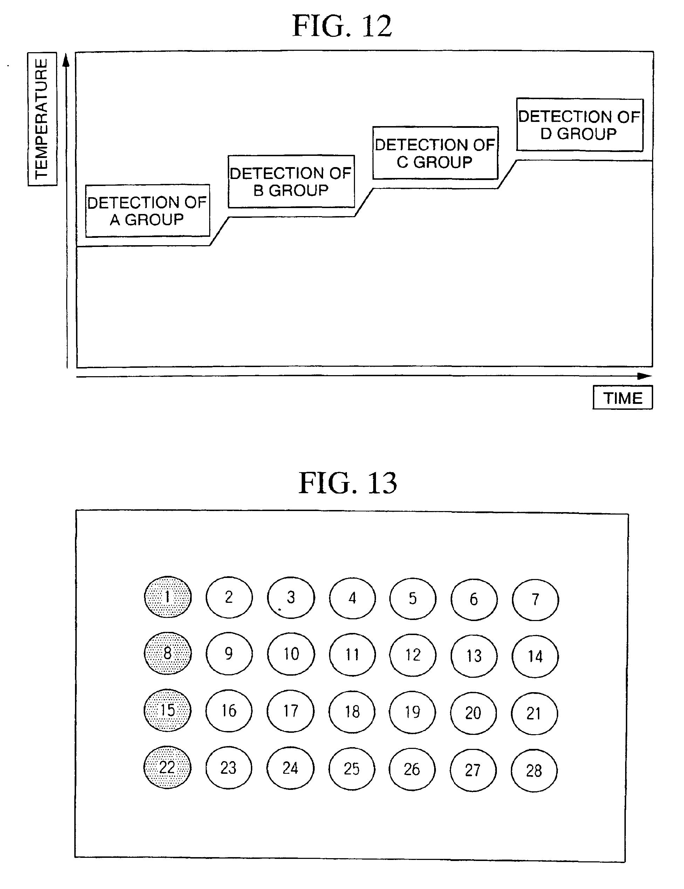

FIG. 8 to FIG. 11 schematically show the change in the hybridization state in experimental systems for detecting four types of sequences. In these drawings, an attempt was being made to detect the sequences of target nucleic acids A, B, C, and D. The relation between the respective Tm values of the target nucleic acids A, B, C, and D (/°C) (Tm(A), Tm(B), Tm(C), Tm(D)) is Tm(A)<Tm(B)<Tm(C)<Tm(D). In the experimental system, the measurement temperature was kinetically changed by a scheme as shown in FIG. 12, in order to detect the target nucleic acids in the respective temperatures.

FIG. 8 shows the hybridization state at the temperature T(A') near Tm(A) which is the lowest temperature among those in FIG. 8 to FIG. 11. The signal intensity of the hybrid between the target nucleic acid A and its perfect matched probe was higher than the signal intensity of hybrids between the target nucleic acid A and its single base mismatched probes, but lower than the signal intensity derived from hybrids including target nucleic acids B, C, and D having higher Tm values.

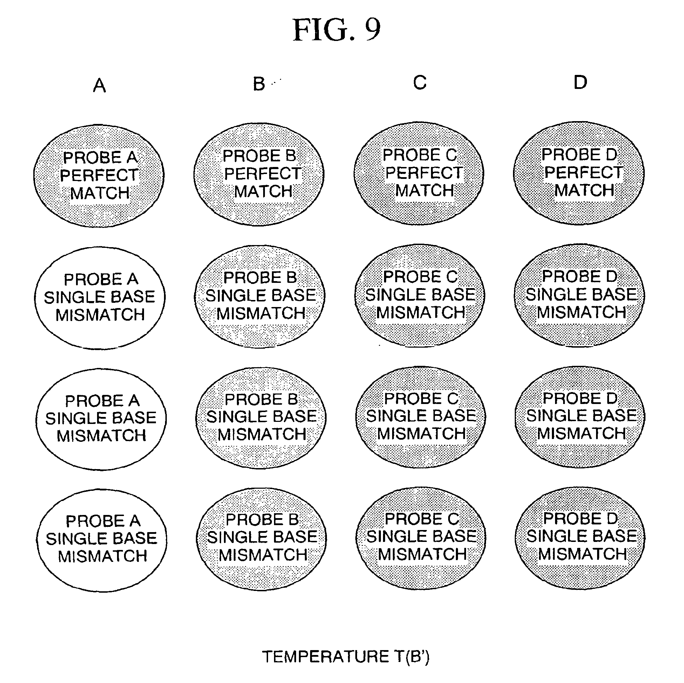

FIG. 9 shows the state where the temperature was further increased from T(A') to T(B') which is near Tm(B). At this temperature, the signal intensity of the hybrid between the target nucleic acid B and its perfect matched probe was higher than the signal intensity of hybrids between the target nucleic acid B and its single base mismatched probes, but lower than the signal intensity derived from hybrids including target nucleic acids C and D having higher Tm values. Furthermore, at this temperature, although the signal of the hybrid between the target nucleic acid A and its perfect matched probes was detected, the signals of the hybrids between the target nucleic acid A and its single base mismatched probes were almost undetected.

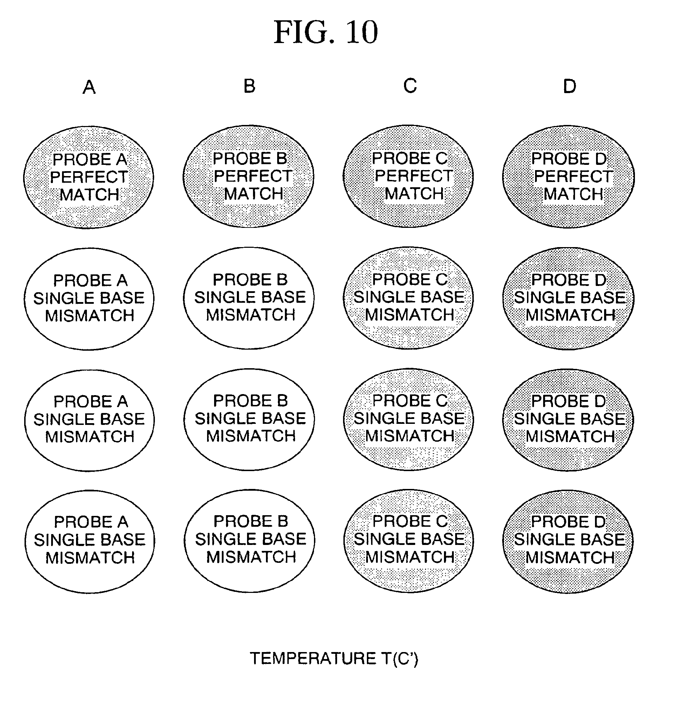

FIG. 10 shows the state where the temperature was further increased from T(B') to T(C') which is near Tm(C). At this temperature, the signal intensity of the hybrid between the target nucleic acid C and its perfect matched probe was higher than the signal intensity of the hybrids between the target nucleic acid C and its single base mismatched probes. Furthermore, at this temperature, the signal of the hybrid between the target nucleic acid A and its perfect matched probe and the signal of the hybrid between the target nucleic acid B and its perfect matched probe were respectively detected. However, the signal intensity of the hybrid between the target nucleic acid A and its perfect matched probe was specifically decreased compared to FIG. 8. Moreover, the signals of the hybrid between the target nucleic acid A and its single base mismatched probes and the signals of hybrids between the target nucleic acid B and its single base mismatched probes were almost undetected.

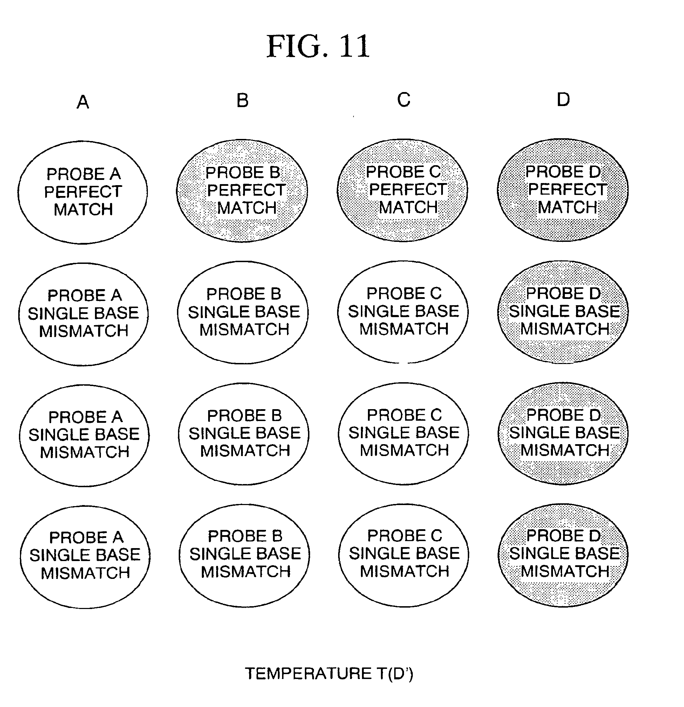

Next, FIG. 11 shows the state where the temperature was further increased from T(C') to T(D') which is near Tm(D). At this temperature, the signal intensity of the hybrid between the target nucleic acid D and its perfect matched probe was higher than the signal intensity of hybrids between the target nucleic acid D and its single base mismatched probes. Furthermore, at this temperature, the signal of the hybrid between the target nucleic acid B and its perfect matched probe and the signal of the hybrid between the target nucleic acid C and its perfect matched probe were respectively detected. However, the signal intensity of a hybrid between the target nucleic acid B and its perfect matched probe was specifically decreased compared to FIG. 9. Moreover, the signals from the hybrids including the target nucleic acid A, the hybrids between the target nucleic acid B and its single base mismatched probes, and the hybrids between the target nucleic acid C and its single base mismatched probes were almost undetected.

The signal data was not obtained at a fixed time point, but kinetically obtained as in the example shown in FIG. 12. More specifically, the time for kinetically obtaining the data differs depending on the conditions of the hybridization reaction. However, generally it ranges from several minutes to several hours in total after starting the hybridization reaction. It is of course also possible to obtain data for the total times outside of this range if the reaction conditions suit. In the example shown in FIG. 12, the temperature was stepwise and intermittently increased. However it is also possible to continuously increase or decrease.

The hybridization between a target nucleic acid and the probes is generally performed under the optimum condition assumed based on the sequence information. Consequently, usually it is not known whether the assumed conditions are really the optimum conditions. Therefore, in a conventional nucleic acid information detection method for measuring statically (non-kinetically) or at a fixed point, if the data is obtained under non optimum conditions, there have been problems of the reliability of the data being insufficient, and irregularities in the data, and so on.

However, in the present invention, by changing the reaction conditions linking with kinetically obtaining the data, it becomes possible to progress the hybridization reaction under a plurality of reaction conditions and measure this with the passage of time. Therefore, for example, even in a case of using a plurality of types of probes having different characteristic values peculiar to the sequence such as the Tm value, if the experiment is performed considering the range of the characteristic values when kinetically obtaining data, it becomes possible to detect a plurality of sequences in a same nucleic acid and a plurality of types of target nucleic acids in a same system approximately at the same time.

In this manner, in the embodiment of the present invention, data may be kinetically obtained, which is different from the conventional method for statically obtaining data based on the fixed time point observation, and a plurality of probes may be used. As a result, it becomes possible to detect a plurality of sequences having different Tm values in the same system accurately at high speed.

An embodiment of the nucleic acid sequence mutation detection method of the present invention is characterized in that the probes are a plurality of types of probes having a plurality of types of sequences and the probes have mutually overlapped sequences.

In the method of this embodiment, identical sequences in a target nucleic acid are detected by a plurality of types of probes. All of these plurality of types of probes share the recognition sequence in the target nucleic acid. That is, they are overlapping in the recognition sequence and are respectively hybridized with bases which mutually differ at least at one base point.

By using such a plurality of types of probes with respect to identical target nucleic acids, it becomes possible to detect the mutation more accurately. In some cases, a probe may have a higher-order structure depending on, the sequence, its length, or the usage condition, causing a fault in the hybridization with the target nucleic acid. If such a probe is only used to detect the target nucleic acid, there may be problems of false positive or false negative. However, if a plurality of types of probes are used, the probability of normal hybridization between at least one type of probe and the target nucleic acid can be expected to be higher. Therefore, it becomes possible to increase the reliability of the detection results.

More specifically, for said probes having a plurality of types of sequences, if using the overlapping probes such as; a perfect matched probe having a perfect complementary sequence at least partially with the target nucleic acid sequence, one or more types of imperfect matched probes having at least one partial mutation in the perfect matched probe, and the perfect matched probe or the imperfect matched probe having an extended or shortened base sequence on both ends or one end, more accurate measurement (determination of mutation) becomes possible.

Preferably, the difference in the Tm values between the short probes and the long probes is arranged in a range from 5°C to 10°C. The measurement is kinetically performed using these probes in the same system. The reason is that, if the difference in the Tm value between the probes is from 5°C to 10°C, the signal from the perfect matched spot with the shorter probe may be expected even at a temperature near the Tm of the longer probe.

A DNA microarray used in the nucleic acid information detection method of the present invention is not specifically limited, and a normal microarray may be used. Examples of usable microarray include a plurality of microarrays provided on a slide tip, that is a plurality of probe spots in respective microarrays. Here, probe spot denotes a minimum unit for fixing a probe.

The size of the slide tip is normally in a range of 0.5 to 20.0cm x 0.5 to 20.0cm x 0.01 to 1.0cm. The size of the microarray is normally in a range of 3.0mm² to 16cm². Furthermore, the probe spot may be approximately circular, approximately rectangular, or polygonal, and the diameter or one side length is normally about several hundreds µm. The number of probe spots in one microarray is normally in a range from 10 to 1000. It is of course possible to use a DNA microarray outside of the range defined above according to experimental conditions.

If using the DNA microarray, it is possible to apply the same conditions to all the probe spots. If using a plurality of probes, it is possible to detect the information of a plurality of nucleic acids at the same time.

Due to the first characteristic of the present invention wherein a signal may be kinetically obtained, it is also of course possible in this embodiment using the DNA microarray, to solve the problem of longer reaction time of the hybridization, which has been a problem in conventional static measurements which do not measure with the passage of time. That is, as described above, the hybridization reaction time may be shortened by starting the hybridization at a temperature lower than the Tm and then serially increasing the reaction temperature.

In a preferred embodiment, the nucleic acid information detection method of the present invention is characterized in that the hybridization between a target nucleic acid and the probes is performed by making a liquid sample including a target nucleic acid contact with a probe fixed onto a porous body.

In this embodiment, the probe is fixed onto a carrier having a porous structure, which is different from a conventional substrate surface. Therefore, the surface area being fixed with the nucleic acid is rapidly increased, causing an increase in the speed and the sensitivity for detecting the nucleic acid information. The porous body used here means any porous body appropriate for fixing the nucleic acid, and is not specifically limited. However, examples includes aluminum oxide film manufactured by anodization, for example, Anodisc (trade name) made by Whatman Co. Ltd.

In another preferable embodiment of the present invention, it is preferable to include performing a process for making a liquid sample including a target nucleic acid, reciprocate once or a plurality of times in said porous body with the respective signal measurement conditions being changed. In this manner, by making the liquid sample including the target nucleic acid reciprocate in the porous body, the probes fixed onto the porous body and the target nucleic acid are mutually contacted more frequently, contributing to further progressing of the reaction and a further increase the sensitivity. Here, the reaction conditions such as reaction temperature are preferably changed linked with the reciprocation process inside the porous body of the sample.

In order to make the sample reciprocate once or a plurality of times in the porous body in this manner, it is necessary to provide a device for forcibly controlling the sample mobilization in and out of the porous part in a reaction carrier for performing the hybridization reaction. However, if the sample is made to reciprocate under a fixed time control using this device, it becomes possible to control the temperature in the reaction system more accurately, causing an advantage of increasing the detection accuracy and obtaining more sensitive data.

In all of the embodiments of the nucleic acid information detection method of the present invention, it is possible to detect the hybrid based on the fluorescent marker. The type of the fluorescent marker used includes FITC, rhodamine, Cy3, Cy5, Texas Red, or the like. However, other types may be used.

The target nucleic acids detectable by the nucleic acid information detection method of the present invention are not specifically limited. However, non-specific examples include the oncogene, the intracellular drug resistance gene, the cell cycle regulator gene, the apoptosis related gene, and the like.

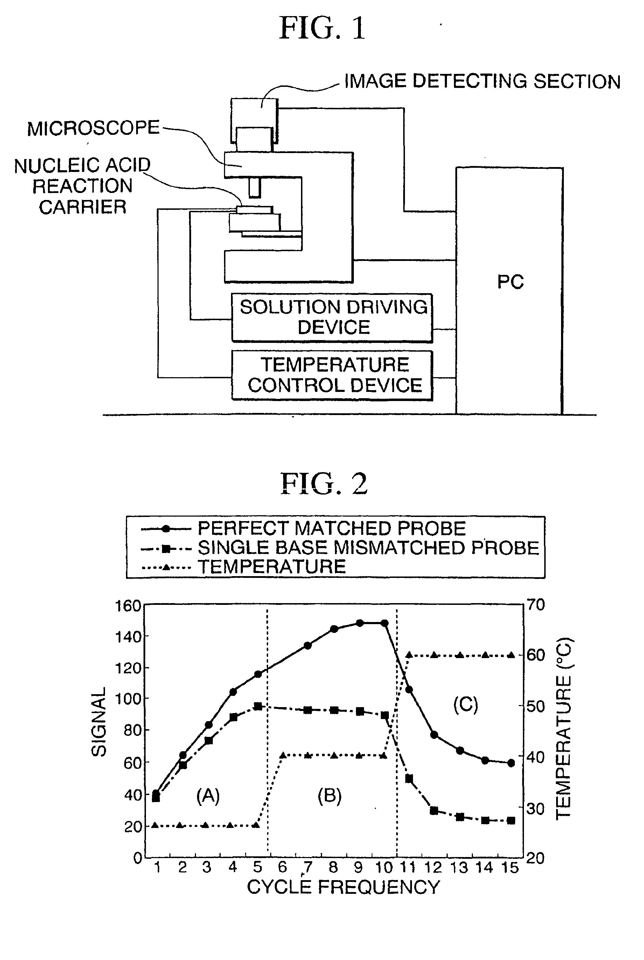

The present invention provides a nucleic acid information analyzer which can be used particularly in the abovementioned nucleic acid information detection method. The analyzer of the present invention is characterized in including: a sample storage vessel for containing a sample including a target nucleic acid; a nucleic acid reaction carrier including a porous structure which can fix the nucleic acid and connected to the vessel; a driving device for mobilizing the sample under control between the vessel and the nucleic acid reaction carrier without leaking; a device for controlling the reaction temperature on the reaction carrier; and a device for detecting a signal from the hybrid between the target nucleic acid and probes formed on the porous structure. The scheme of an embodiment of this device is as shown in FIG.1. The device shown in FIG. 1 is a detection apparatus based on a fluorescence microscope, and operation of the respective components is controlled by PC.

Here, the sample storage vessel (not shown in FIG. 1) is not specifically limited, as long as it is suitable for storing sample nucleic acids. Moreover, the nucleic acid reaction carrier including a porous structure which can fix the nucleic acids is not specifically limited, as long as the material is suitable for fixing the nucleic acids and does not inhibit the hybridization. However, an specific example of the nucleic acid reaction carrier includes the abovementioned DNA microarray. Particularly it is preferable that the carrier having a porous structure is attached with a solid phase of a desired nucleic acid probe. Here, the structure between the sample storage vessel and the nucleic acid reaction carrier is preferably sealed in order to mobilize the sample nucleic acid to reciprocate to the porous structure once or a plurality of times without leaking. Furthermore, it is preferable to have enough capacity to retain the whole amount of the sample nucleic acid. A kind of pump suitable for transferring µl unit solutions may be used for the solution driving device for mobilizing the sample solution between the sample storage vessel and the nucleic acid reaction carrier. The temperature control device which controls the reaction temperature on the nucleic acid reaction carrier is preferably able to set the temperature in about 0.1°C units, and preferably able to set the temperature of not only the nucleic acid reaction carrier but also the environmental temperature of the sample storage vessel, the passages between the sample storage vessel and the nucleic acid reaction carrier to the same temperature. The device for detecting the signal from the hybrid may be, for example in the case where the hybrid is labeled with a fluorescent substance, a microscope comprising a CCD camera (image detecting section) for imaging optical signals from the nucleic acid reaction carrier. It is desirable that the microscope, the image detecting section, the solution driving device, and the temperature control device are appropriately controlled by a PC. The construction is such that, the obtained image is converted into signal information of respective probes by arithmetic processing of the PC. Then the absolute value, the relative value, the amount of change are arithmetically processed with a parameter such as the reaction temperature, frequency of the drive, the time, and the like so that the definitive gene mutation information can be displayed.

In one aspect of the nucleic acid information analyzer of the present invention, the apparatus preferably further includes one or more solution storage vessels connected to said nucleic acid reaction carrier for storing different types of solutions from the sample solution including the target nucleic acid, and a device which appropriately mixes the various solutions contained in the solution storage vessel and sends these to said nucleic acid reaction carrier.

In this device, by providing one or more solution storage vessels connected to the nucleic acid reaction carrier for storing different types of solutions from the sample solution including the target nucleic acid, it becomes possible to progress the hybridization reaction while changing the reaction conditions rather than the reaction temperature, such as the composition or pH of salt, additives, or the like in the reaction solution. Moreover, if there is a device which mixes the plurality of solutions contained in the solution storage vessel and sends these to said nucleic acid reaction carrier, it is possible to provide a gradient of the salt concentration or pH in order to change the reaction conditions.

The type of nucleic acid which can be analyzed in the nucleic acid information analyzer of the present invention, is not specifically limited. However, examples of nonrestrictive target nucleic acid include the oncogen, the intracellular drug resistance gene, the cell cycle regulator gene, the apoptosis related gene, and the like.

### (Example 1)

### Method

Four types of oligo DNA represented by SEQ ID NO:1 to NO:4 were prepared for the probes and solid-phased onto an aluminum oxide substrate manufactured by anodization. For verifying the reaction, the hybridization reaction was performed using an oligo DNA represented by SEQ ID NO:5 as a sample. The 3' terminus of this oligo DNA was labeled with FITC. The oligo DNA (SEQ ID NO:5) was complementary to the probe (SEQ ID NO:1). The probes (SEQ ID NO:2 to NO:4) had single base mutations on the middle base with respect to the complementary strand of the oligo DNA (SEQ ID NO:5).

The oligo DNA sample was diluted with a 1XSSPE buffer solution to make a 10nM concentration, and then hybridization reactions were performed with the respective probes on the substrate. The temperature of the nucleic acid reaction carrier was controlled linked with the mobilization of the solutions to the substrate being a nucleic acid reaction carrier. The specific conditions were as follows.

| Cycle frequency | Temperature |
|---|---|
| 1-5 | 25°C |
| 6-10 | 40°C |
| 11-15 | 60°C |

The respective cycle took 1 minute for reciprocating the solution between the sample storage vessel and the nucleic acid reaction carrier.

### Results

The results are shown in FIG. 2. The graph shows the detection signals with respect to the probes (SEQ ID NO:1 and NO:2). The region (A) in FIG. 2 shows the results of the hybridization performed at 25°C. The signal intensity was increased as the mobilization cycle frequency of the solutions was increased. Here, a non specific reaction between the target nucleic acid and the probes was generated, causing an effect to promote the hybridization. However, since it was a non specific reaction, the signal difference was small between the perfect matched probe (SEQ ID NO:1) and the imperfect matched probe (SEQ ID NO:2). The region (B) shows the results when the reaction temperature was 40°C. The signal derived from the probe (SEQ ID NO:1) hybrid continued to increase, while the signal intensity derived from the probe (SEQ ID NO:2) was slightly decreased. In this manner, by detecting at the stage when a signal intensity difference is generated between the perfect matched probe and the imperfect matched probe, due to the large difference in the brightness, it becomes possible to detect the nucleic acid sequence more accurately. Here, the signal intensity derived from the probe (SEQ ID NO:1) hybrid appears most strongly. The signals derived from the probe (SEQ ID NO:3 and NO:4) hybrids showed similar results to the probe (SEQ ID NO:2) hybrid (not shown). Therefore, it was verified that a single base mutation could be accurately detected by the hybridization reaction which controls the temperature.

### (Example 2)

### Method

An experiment was performed to detect a mutation in the p53 gene derived from a cell line. The cell line from the human lymphoblastic glomus tumor WTK1 was used for the sample. It has been confirmed that this cell line has a mutation from ATG to ATA at the codon 273 on the exon 7 of the p53 gene. The 98bp including this mutation portion was amplified by PCR. In the PCR, 5' terminus of the primer was labeled with FITC in order to label the amplified product with fluorescence. The amplified DNA was dissolved in water and denatured by heat at 95°C for 10 min. It was quickly cooled after 10 min, and dissolved in a buffer solution for hybridization in order to make single strand DNA. The amplified sequence was represented by SEQ ID NO:6, and the detected sequence was represented by SEQ ID NO:7.

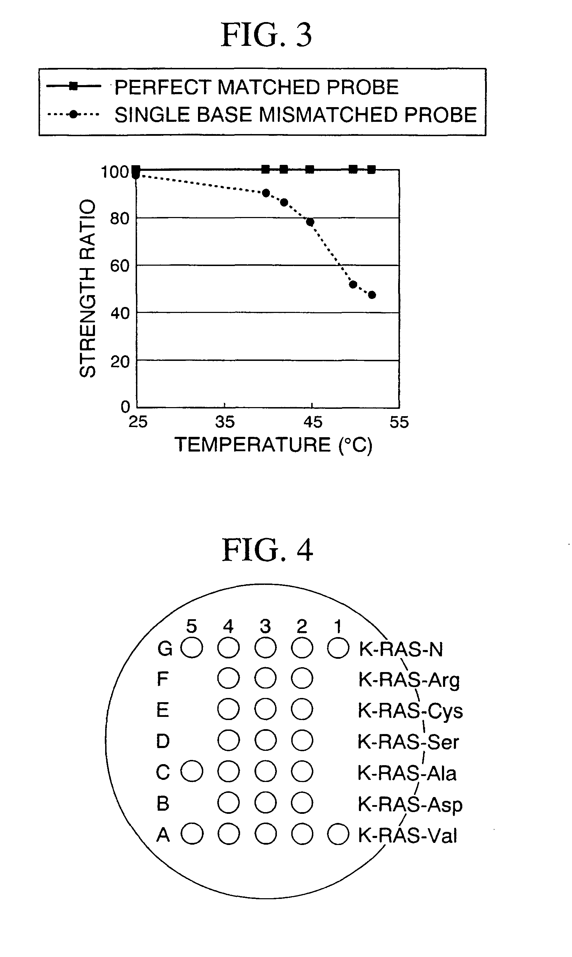

On the other hand, for detecting the mutation, a plurality of probes having the assumed mutation portion in the middle were prepared and solid-phased on a substrate having a porous structure. The solid-phased probes are represented by SEQ ID NO:8 to NO:11. Here the probe (SEQ ID NO:10) is complementary to SEQ ID NO:7. The hybridization reaction using these samples was performed linked with changing the temperature, and the sample solutions were further put into and taken out from the nucleic acid reaction carrier having the porous structure (drive control). Among these reactions, the results for the signal information derived from the hybrids formed by probes (SEQ ID NO:10 and NO:11) are shown in FIG. 3. This shows the signal intensity derived from the probe (SEQ ID NO:10 and NO:11) hybrids represented by relative intensity ratio if the signal intensity derived from the probe (SEQ ID NO:10) hybrid is 100.

### Results

The difference in the signal intensity ratio between the perfect matched probe and the imperfect matched probe including the single base mismatch with the perfect match, was increased as the temperature was increased. Therefore, it was confirmed that a single base mutation undetectable at room temperature, could be identified by controlling the temperature.

### (Example 3)

### Method

A method for determining the presence/absence of mutation in the neighborhood of Codon12 in K-Ras oncogene is shown. On a microarray, seven types of K-Ras oncogene probes having mutations inserted in the Codon12 (SEQ ID NO:15-21) were spotted in the arrangement shown in FIG. 4. Then, the Tm between this microarray and sample K-Ras genes labeled with fluorescence (amplified by PCR using primers (SEQ ID NO:12 and 13)) was measured. The experimental step used at this time is described hereunder. The experimental step comprises four steps of 1) preparation of fluorescence labeled samples, 2) preparation of the microarray, 3) hybridization of the samples with respect to the microarray, 4) data analysis.

### 1) Preparation of fluorescence labeled samples:

The target gene set was amplified and labeled with fluorescence. The samples used at this time are not specifically limited as long as they are a part of the human body. However, a tissue section sampled from cancerous tissue, a cellular section obtained from a microdissection method, a cultured cell, or the like are mainly used. In the present embodiment, the human K-Ras gene template set (Cat#7242) available from Takara Shuzo Co., Ltd. was used. With respect to seven types of templates, the genes were amplified using the PCR kit (Cat#7112) which can amplify the K-Ras gene Codon12 in the same manner. At this time, anti-sense primers having 5' terminus labeled with FITC fluorescent marker as represented by SEQ ID NO:12 and NO:13 were used. After the PCR, electrophoresis was performed using an agarose gel formed from 3%NuSieve(FMC) to confirm the amplified products. The amplified samples obtained were repeatedly treated with the Asymetrix PCR method in order to enhance the fluorescent labeling. In the Asymetrix PCR method, the composition and the temperature cycle from the first PCR method was used, with the sense strand primer removed. 3M ammonium acetate (Wako) was added to the PCR products for making 10%(V/V), and ethanol was further added for making 70% concentration. The PCR products were ventilated the whole day and night at 20°C, and then precipitated by centrifugation at 12,000rpm x 2 min. The precipitation was washed with 70% ethanol twice, and then dehydrated by a SpeedVac (Savant Co. Ltd,.)

### 2) Preparation of microarray:

Regarding the base sequence of K-Ras gene, referring to a database such as the GenBank, probes (SEQ ID NO:15 to 21) were solid-phased onto the microarray. In order to make the layout shown in FIG. 4, the composed probes were dispensed using a micro dispersion system using a piezo device. In FIG. 4, negative control probes (SEQ ID NO:14) labeled with fluorescence (for referring to the spot positioning) were arranged at the spots A1, A5, C5, G1, and G5. Probes corresponding to K-RAS-Val mutant (SEQ ID NO:15) were arranged at the spots A2 to A4. Probes corresponding to K-RAS-Asp mutant (SEQ ID NO:16) were arranged at the spots B2 to B4. Probes corresponding to K-RAS-Ala mutant (SEQ ID NO:17) were arranged at the spots C2 to C4. Probes corresponding to K-RAS-Ser mutant (SEQ ID NO:18) were arranged at the spots D2 to D4. Probes corresponding to K-RAS-Cys mutant (SEQ ID NO:19) were arranged at spots E2 to E4. Probes corresponding to K-RAS-Arg mutant (SEQ ID NO:20) were arranged at the spots F2 to F4. Probes corresponding to K-RAS-N natural sequence (SEQ ID NO:21) were arranged at the spots G2 to G4.

### 3) Real time hybridization monitoring:

Pure water was added to the dehydrated ethanol precipitate so that it dissolved well in order to make the fluorescence labeled samples. For the solutions for hybridization, the samples were suspended in a 3 x SSPE (sodium phosphate buffer solution, refer to a technical data: "DNA Microarray and the Latest PCR Method", Cell Technology additional volume Genome Science Series 1, published by Shujun-Sha), and 10% (V/V) ExpressHyb (Clontech Co. Ltd.,) in order to make a 10% solution. The hybridization was analyzed by an experimental system where the BX-51TFR made by Olympus Co. Ltd., was connected with a cooled CCD camera. This experimental system was designed to enable automation of driving the solution around the reaction filter, controlling the temperature, and recording the image of the fluorescent spots. A 50µL of reaction solution was added to the reaction part in the exclusive chamber installed with the microarray of 6mm diameter, and then the solution was driven, the temperature changed, and the fluorescent image taken.

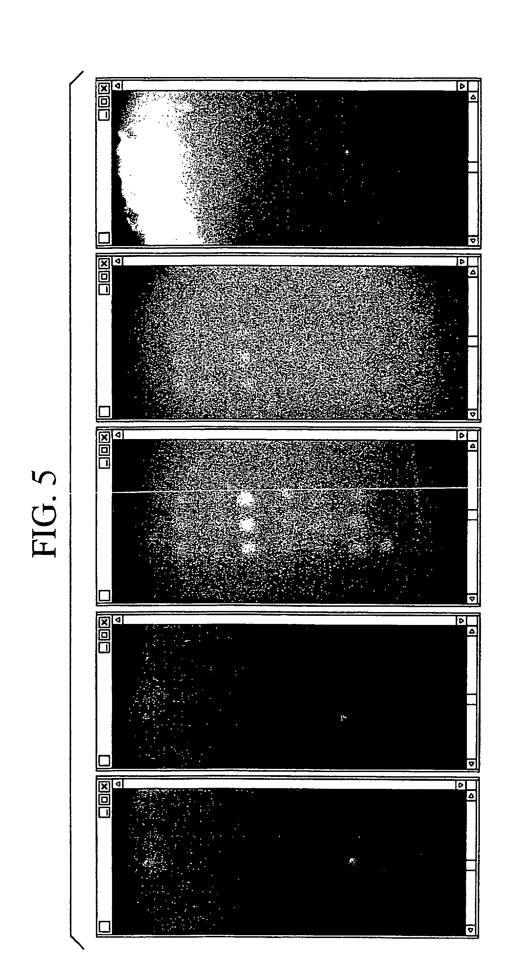

The experimental results obtained by using fluorescence labeled samples derived from the template are shown. A PCR product having a normal K-Ras gene sequence was used as the control sample. FIG. 5 shows the time-varying fluorescent spots on the microarray obtained with changing the temperature when hybridizing. FIG. 5 shows the fluorescent images, respectively from the bottom, for 1min, 10min, 20min, 30min, and 40min after hybridization, which were programmed so as to accompany the driving of the solution and to change the temperature of the solutions. The temperature of the reaction chamber at room temperature at the beginning was set so as to give 72°C at the end. Obviously from the figure, the fluorescent intensity for most spots was increased immediately after the reaction. Furthermore, as the temperature was increased, the Cys spots corresponding to E2, E3, and E4 in the figure (sequence TGT on Codon12) showed the highest level of the fluorescence after 20 to 30min. In such thermal environment, it was found that, among the solid-phased seven types of probes having different base sequences, the sample was bound to the probe having the lower calculated Tm value more strongly than the probe having a perfectly complementary sequence with the sample. Furthermore, by increasing the temperature in the reaction chamber, the fluorescence intensity of the Cys spots (E2 to E4 spots) was gradually decreased. At the step of 72°C temperature, only the fluorescent spots of Gly ((G2 to G4, sequence GGT on Codon12) that were an actual perfect match with respect to the target nucleic acid, were observed (after 40min). The series of images was saved in a hard disk together with information such as the temperature conditions.

### 4) Analysis:

The obtained images were analyzed using an analysis software which was programmed for detecting mutations. Based on the information on the fluorescent images obtained as the experimental results, the Tm value and the degree of affinity between the target nucleic acid and the probes can be determined. As a result, from the 3) experiment, it was found that the degree of hybridization between the probe of the sample K-Ras codon 12 and the target nucleic acid varies depending on environmental factors, and the mismatched probes may have rather higher affinity than the perfect matched probe in some temperature conditions. Moreover, it can be intuitively understood from the images that the affinity between the probes and the sample differs depending on the mismatched base sequence. In this manner, it was shown that the real time affinity analysis and the hybridization monitoring between a sample and a plurality of probes may be performed in one reaction array. Using this experimental system, it can be expected to determine the actual Tm value and to obtain new findings on the stability of DNA double strands. Actually, by analyzing the affinity to probes having various mutations, or by measuring the transcription efficiency of the promoter part, it may be utilized for analyzing the hybridization mechanism more accurately.

### (Example 4)

An experimental step performed based on the invention for examining the p53 tumor suppresser gene and the K-Ras oncogene at the same time is described hereunder. The experimental step comprises four steps of 1) preparation of fluorescence labeled samples, 2) preparation of the microarray, 3) hybridization with respect to the DNA tip, 4) data analysis.

### 1) Preparation of fluorescence labeled samples:

The target gene set was amplified and labeled with fluorescence. The samples used at this time are not specifically limited as long as they are a part of the human body. However, a tissue section sampled from cancerous tissue, a cellular section obtained from a microdissection method, a cultured cell, or the like are mainly used. In the example, squamous cells collected from a normal human intraoral were used. The cell fragment suspension was obtained by gargling with saline solution dissolved with 1M NaCl several times, and then gargling with PBS one more time. The cell suspension was precipitated by centrifugation at 2,000 rpm x 10 min, and suspended a cell lysate into PBS adjusted to 0.2 µg/mL Protease K(Wako) and 0.1%SDS (sodium dodecyl sulfate). The sample contained cell lysate was reacted for 30 min at 37°C, and then heat-treated for 30 min at 95°C so as to deactivate the Protease K. The reacted sample was moved into a 1.5 mL Eppendorf tube, and centrifugal separation at 12,000 x 2 min was carried out so as to precipitate the non-dissolved cell fragment. This centrifugal supernatant was used as the nucleic acid extract. The nucleic acid extract obtained was suspended into a PCR master mix, and 50 cycles of PCR reaction were carried out. The master mix was formulated using the PCR Core kit 1 of Takara Shuzo Co., Ltd. and the primer pairs shown in SEQ ID NO:22 to 23 and SEQ ID NO:24 to 25 according to the instructions of the kit After the PCR, electrophoresis was performed using an agarose gel formed from 3%NuSieve(FMC) to confirm the amplified products. The amplified samples obtained were repeatedly treated with an Asymetrix PCR method in order to enhance the fluorescent labeling of the nucleic acid. The Asymetrix PCR method comprises the composition and the temperature cycle from the first PCR method with the sense strand primer removed. 10% 3M ammonium acetate (Wako) was added to the sample treated with Asymetrix PCR, and ethanol was further added for making 70% concentration. The PCR products were ventilated the whole day and night at 20°C, and then precipitated by centrifugation at 12,000rpm x 2 min. The precipitation was washed with 70% ethanol twice, and then dehydrated by a SpeedVac (Savant Co. Ltd,).

### 2) Preparation of microarray:

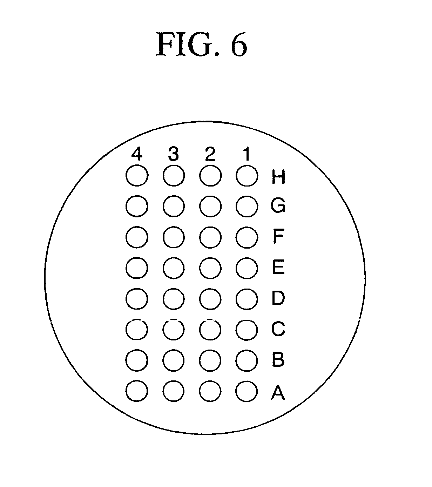

For the base sequences of the probes used for the detection tips of the P53 gene and K-Ras gene a database such as the GenBank was referenced. In order to make the layout shown in FIG. 6, the composed probes (SEQ ID NO:26-53) were dispensed using a micro dispersion system using a piezo device. In FIG. 6, probes (SEQ ID NO:26 to 28) were respectively arranged at the spots A1 to A3. A negative probe (SEQ ID NO:29) labeled with fluorescence (for referring to the spot positioning) was arranged at the spots A4. Probes (SEQ ID NO:30 to 33) were respectively arranged at the spots B1 to B4. Probes (SEQ ID NO:34 to 36) were respectively arranged at the spots C1 to C4. No probe was arranged at spot C4. Probes (SEQ ID NO:37 to 40) were respectively arranged at the spots D1 to D4. Probes (SEQ ID NO:41 to 44) were respectively arranged at the spots E1 to E4. Probes (SEQ ID NO:45 to 48) were respectively arranged at the spots F1 to F4. Probes (SEQ ID NO:49 to 51) were respectively arranged at the spots G1 to G4. No probe was arranged at the spot G4. The same probes (SEQ ID NO:29) were respectively arranged at the spots H1 and H4. Probes (SEQ ID NO:52 to 53) were respectively arranged at the spots H2 and H3. Probes for K-ras were arranged in the arrays from A to D except for the marker spots. Probes for P53 were arranged in the arrays from E to H except for the marker spots.

### 3) Hybridization:

50 µl of pure water was added to the dehydrated ethanol precipitate so that it dissolved well in order to make the fluorescence labeled samples. For the solutions for hybridization, the samples were suspended in a 3 x SSPE (sodium phosphate buffer solution, refer to technical data: "DNA Microarray and the Latest PCR Method", Cell Technology additional volume Genome Science Series 1, published by Shujun-Sha), and 10% (V/V) ExpressHyb (Clontech Co. Ltd.,) in order to make a 10% solution. The hybridization was analyzed by an experimental system where the BX-51TFR made by Olympus Co. Ltd., was connected with a cooled CCD camera. This experimental system was designed to enable the automation of driving the solution around the reaction filter, controlling the temperature, and recording the image of the fluorescent spots.

### Analysis results

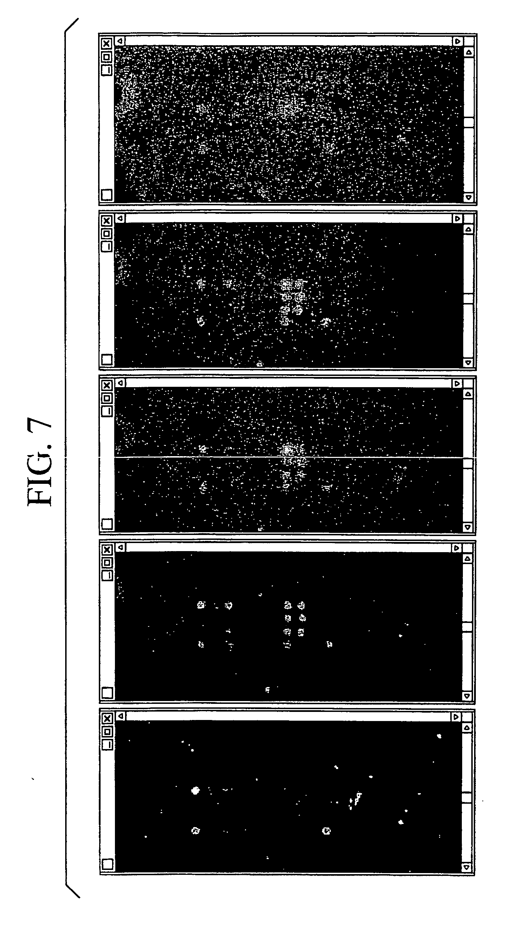

The experimental results obtained by using samples from a normal human are partly shown. The time-varying fluorescent spots of the DNA tips obtained by the experimental equipment are shown in FIG. 7. FIG. 7 shows the fluorescent images, respectively from the bottom, for 1min, 10min, 20min, 30min, and 40min after hybridization. The temperature of the reaction chamber at room temperature at the beginning was set so as to give 72°C at the end. Obviously from the figure, the fluorescent intensity for most spots was increased immediately after the reaction. Furthermore, as the temperature was increased, only one P53 spot in the position at the top in the figure showed a detectable level of the fluorescence (after 30min). In such an environment, since the Tm value of the K-Ras gene with respect to the solid-phased probe was high, the brightness of the most K-Ras fluorescent spot was not clearly different. Therefore, by further increasing the temperature of the reaction chamber, the brightness of the K-Ras gene spots could be clearly determined (after 40min). The series of images was saved in a hard disk together with information such as the temperature conditions.

### 4) Analysis:

The obtained images were analyzed using an analysis software which was programmed for detecting mutations. Based on the base sequence of the probe spotted on the microarray used and the information on the images obtained as the experimental results, the analysis software can automatically determine the base sequence of the samples. By analyzing the images obtained from the results of the 3) experiment, it became clear that the base sequence of P53exon7 of the sample was as represented by SEQ ID NO:54. Moreover, it became clear that the base sequence including K-RasCodon12 was as represented by SEQ ID NO:55. From this it was seen that the base sequences of both P53 and K-Ras samples were normal. In this manner, it was shown that the sequences of two genes can be promptly analyzed in one reaction array.

### (Example 5)

Next is an example of a case where the method of the present invention was applied for analyzing the gene expression. The mRNA respectively extracted from a normal tissue and a pathological tissue were labeled with fluorescent marker by reverse transcription reaction to compose single strand cDNAs. The microarray of the samples was set in the apparatus shown in FIG. 1 which can kinetically obtain data so that the hybridization reaction was performed with respect to cDNA expressed from the respective tissues. A CCD camera was used for obtaining the image data. Regarding the CCD camera, although it is necessary to set an appropriate exposure time according to the brightness of the photographic subject, the brightness of the gene hybridization images obtained may vary depending on the degree of the fluorescence labeling of the sample, the quantum efficiency of the fluorescent substance itself, the reaction efficiency of the hybridization, and the like. For accurate detection, it is necessary to keep the illuminant image of the spot within an appropriate range in the CCD dynamic range. If it is not appropriate, the image would be darkened, or be over the imaging pickup range, so that accurate detection could be performed. Without previously setting such uncertain image pickup conditions, the reaction was progressed while kinetically obtaining data, confirming the image, and setting the optimum image pickup conditions. The maximum brightness of the obtained hybridization image was adjusted to be about 80% of the CCD dynamic range and the obtained image was analyzed. Consequently it became possible to accurately detect the hybridization image.

### (Example 6)

In order to verify the system of the present invention, hereunder is an outline of the method using a plurality of types of probes having different Tm values made by changing the length of the sequences, in order to determine the presence/absence of mutations in K-ras oncogene Codon12.

On a microarray, sense strands of seven types of 20mer K-ras oncogene probes having mutations inserted in the K-ras Codon12 sequence (SEQ ID NO:56 to 62), and anti-sense strands thereof (SEQ ID NO: 63 to 69), and sense strands of seven types of 17mer K-ras oncogene probes such that single or dibasic were deleted at both terminuses in the above K-ras oncogene probes (SEQ ID NO:70 to 76), and anti-sense strands (SEQ ID NO: 77-83) were spotted in an arrangement shown in FIG. 13.

The Tm value of the 20mer probe group was from 56°C to 58°C, while the Tm value of the 17mer probe group was from 47°C to 49°C, being lower than that of the 20mer probes. The respective probes on this microarray and the fluorescence labeled K-ras oncogene (amplified by PCR using primers which amplify the K-ras Codon12) were hybridized. The fluorescent intensity from the hybrid was measured in an experimental system where the BX-52TRF made by Olympus Co. Ltd., was connected with a cooled CCD camera.

The experiment shown in outline above comprises four main steps of 1) preparation of fluorescence labeled samples, 2) preparation of the microarray, 3) hybridization of the samples with respect to the microarray, and 4) data analysis. Hereunder is a description of the details of the respective steps.

### 1) Preparation of fluorescence labeled samples:

The target gene set was amplified and labeled with fluorescence. Examples of the target sample sources include for example a part of human body. However, these more specifically include a tissue section sampled from cancerous tissue, a cellular section obtained from a microdissection method, a cultured cell, or the like. In the present embodiment, the human K-ras gene template set (made by Takara Shuzo Co., Ltd., Cat#7242) was used. With respect to seven types of templates, the genes were amplified using the PCR kit (made by Takara Shuzo Co., Ltd., Cat#7112) which can amplify the K-ras gene Codon12 in the same manner. For the amplification, primers having 5' terminuses labeled with FITC fluorescent marker were used. After the PCR, electrophoresis was performed using an agarose gel formed from 3%NuSieve(FMC) to confirm the amplified products.

### 2) Preparation of microarray:

For the base sequence of K-ras oncogene, a database such as the GenBank was referenced, and probes (SEQ ID NO:56 to 62, 63 to 69, 70 to 76, 77 to 83) were solid-phased onto the microarray. In order to make the layout shown in FIG. 13, the composed probes were spotted using a micro dispersion system using a piezo device. At the spot 1 in FIG. 13, a 20mer sense strand probe corresponding to K-ras natural sequence (Wt) (SEQ ID NO:56) was arranged. At the spot 2, a 20mer sense strand probe corresponding to K-rasArg mutant (SEQ ID NO:57) was arranged. At the spot 3, a 20mer sense strand probe corresponding to K-rasCys mutant (SEQ ID NO:58) was arranged. At the spot 8, a 20mer anti-sense strand probe corresponding to K-rasWt (SEQ ID NO:63) was arranged. At the spot 15, a 17mer sense strand probe corresponding to K-rasWt (SEQ ID NO:70) was arranged. At the spot 22, a 17mer anti-sense strand probe corresponding to K-rasWt (SEQ ID NO:77) was arranged.

### 3) Hybridization:

For the solutions for hybridization, 40µl of samples after the PCR were suspended into 10µl of 1.25 x SSPE (sodium phosphate buffer solution, refer to technical data: "DNA Microarray and the Latest PCR Method", Cell Technology additional volume Genome Science Series 1, published by Shujun-Sha), in order to make hybridization samples. The hybridization was analyzed by an experimental system where the BX-52TRF made by Olympus Co. Ltd., was connected with a cooled CCD camera. This experimental system was designed to enable the automation of driving the solution around the reaction filter, controlling the temperature, and recording the image of the fluorescent spots. A 50µL of reaction solution was added to the reaction part in the exclusive chamber installed with the microarray of 6mm diameter, and then the solution was driven, the temperature changed, and the fluorescent image taken.

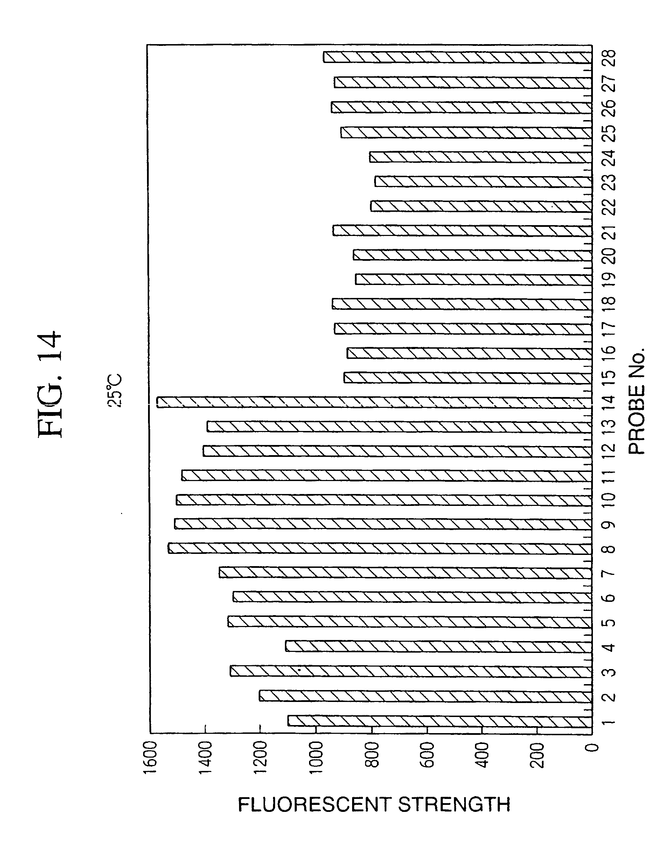

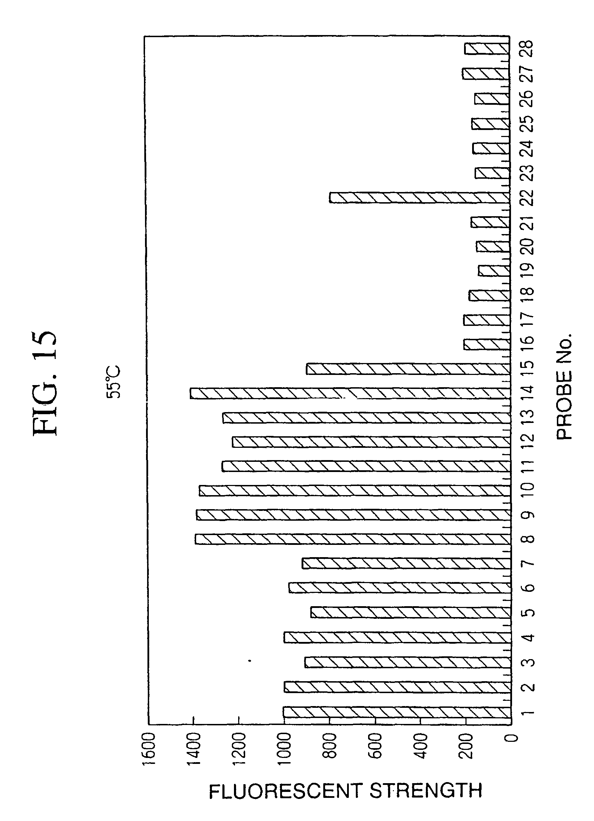

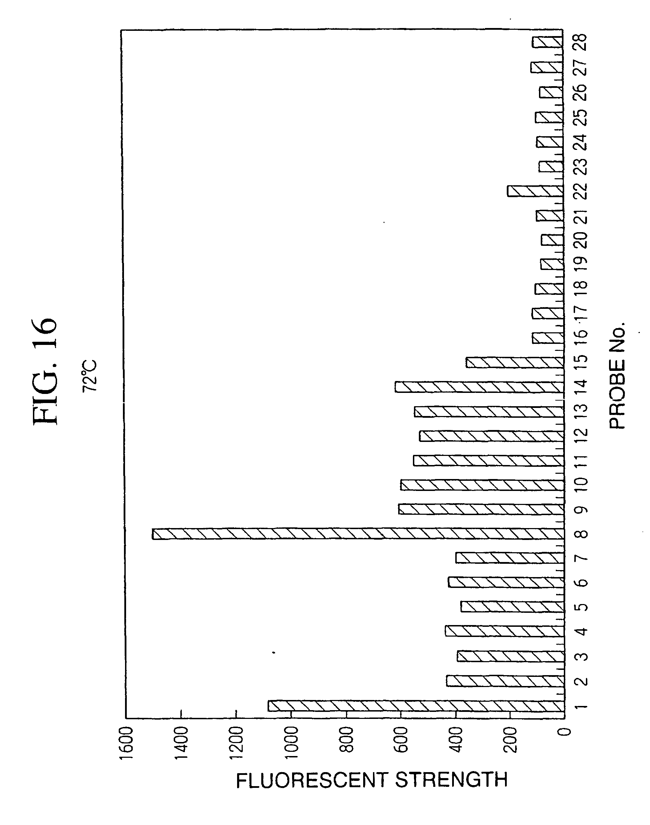

The experimental results obtained by using fluorescence labeled samples derived from the template are shown. A PCR product having a normal K-ras oncogene sequence was used as the control sample. FIG. 14 to FIG. 16 show the time-varying fluorescent spots on the microarray obtained with changing the temperature when hybridizing. This experimental system was programmed so as to accompany the driving of the solution and to change the temperature of the solutions. The temperature of the reaction chamber at room temperature (25°C) at the beginning was so as to give 55°C, and then further so as to give 72°C at the end. Obviously from the figure, the fluorescent intensity for most spots was increased immediately after the reaction. In such a thermal environment, for both the solid-phased 17mer group and the 20mer group, among seven types of probes having different base sequences, high fluorescent intensity was shown at other spots rather than the probe having a perfectly complementary sequence with the sample (FIG. 14). Furthermore, by increasing the temperature in the reaction chamber to 55°C, the fluorescence intensity of the mismatch spots (spots 16 to 21 and 23 to 28) was gradually decreased in the probes in the 17mer group. The only fluorescent spots of Gly (15 and 22, sequence GGT on Codon12) that were actual perfect matches with respect to the target nucleic acid, were observed (FIG. 15). However, the respective probes in the 20mer group were still lower than the Tm values of the probes, and the signal intensity and the perfect match was not yet consistent. Furthermore, by increasing the temperature of the reaction chamber to 72°C, the fluorescence intensity of the mismatch spot was decreased even in the 20mer group. Only the fluorescent spots of Gly (1 and 8, sequence GGT on Codon12) that were actual perfect matches with respect to the target nucleic acid, were observed (FIG. 16). The series of images was saved in a hard disk together with information such as the temperature conditions.

### 4) Data Analysis:

The obtained images were analyzed using an analysis software which was programmed for detecting mutations. Based on the information on the fluorescent images obtained as the experimental results, the Tm value and the degree of affinity between the target nucleic acid and the probes can be determined. As a result, from the 3) experiment, it was found that the degree of hybridization between the probe of the sample K-ras Codon 12 and the target nucleic acid varies depending on environmental factors, and the mismatched probes may have rather higher affinity than the perfect matched probe in some temperature conditions. Moreover, it can be intuitively understood from the images that the affinity between the probes and the sample differs depending on the mismatched base sequence. In this manner, it was shown that the real time affinity analysis and the hybridization monitoring between a sample and a plurality of probes may be performed in one reaction array. Using this experimental system, it can be expected to determine the actual Tm value and to obtain new findings on the stability of DNA double strands. Actually, by analyzing the affinity to probes having various mutations, or by measuring the translation efficiency of the promoter part, it can be utilized for analyzing the hybridization mechanism more accurately.

### INDUSTRIAL APPLICABILITY

If the method and the apparatus of the present invention is used, it becomes possible to obtain lots of more accurate and more reliable information on nucleic acid. Furthermore, it becomes possible to detect a plurality of different sequences in the same system accurately at high speed.

## Claims

1. A nucleic acid information detection method wherein a target nucleic acid and probes having a complementary sequence with at least a portion of said target nucleic acid sequence are contacted with each other in order to form hybrids between said target nucleic acid and said probes, and the amount of signal generated depending on the amount of hybrids is measured in order to detect the information on the target nucleic acid,
said method including kinetically obtaining data of said signal.

2. A nucleic acid information detection method according to claim 1, wherein obtaining the data of said signal is performed while changing a measurement condition or a detection condition of a reaction.

3. A nucleic acid information detection method according to claim 2, wherein obtaining the data of said signal is performed while changing at least one of; a reaction temperature, a composition, a volume, and a type of reaction solution.

4. A nucleic acid information detection method according to claim 3, wherein said change is for the reaction temperature.

5. A nucleic acid information detection method wherein a perfect matched probe having a perfect complementary sequence with respect to at least part of a target nucleic acid sequence, and one or more types of imperfectly matched probes having at least one part of the perfect matched probe mutated are contacted with said target nucleic acid in order to form hybrids between said target nucleic acid, and said perfect matched probe or said imperfect matched probes, so that the information on the target nucleic acid can be detected based on a difference in binding strength of the hybrids,
said method including kinetically obtaining data of said signal while changing continuously or stepwise the condition for measuring or detecting the signal from said hybrids.

6. A nucleic acid information detection method according to claim 5, wherein obtaining the data of said signal is performed while changing at least one of; a reaction temperature, a composition, a volume, and a type of reaction solution.

7. A nucleic acid information detection method according to claim 6, wherein said change is for the reaction temperature.

8. A nucleic acid information detection method according to claim 7, wherein said change of the reaction temperature is to increase the temperature from a temperature lower than a Tm value of the hybrids to be detected to a temperature higher than the Tm value.

9. A nucleic acid information detection method according to claim 7, wherein said change of the reaction temperature is a temperature cycle of one or more times comprising increase and decrease between a temperature lower than the Tm value to a temperature higher than the Tm value.

10. A nucleic acid information detection method according to either claim 8 or claim 9, including a step of measuring a maximum value of the signal intensity while increasing said temperature.

11. A nucleic acid information detection method according to either claim 8 or claim 9, including a step of measuring an amount of change in the signal intensity while increasing said temperature.

12. A nucleic acid information detection method according to any one of claim 5 through claim 11, further comprising the steps of continuously or stepwise increasing the temperature at which a signal from said hybrid is measured, measuring the change in the signal intensity from said hybrid between respective temperatures, and maintaining the temperature when the amount of change starts to decrease.

13. A nucleic acid information detection method according to any one of claim 1 through claim 12, wherein in an identical system where identical reaction conditions are applicable, a plurality of types of probes are used in order to detect the information on a plurality of types of nucleic acids at the same time.

14. A nucleic acid information detection method according to any one of claim 1 through claim 13, wherein said probes are a plurality of types of probes having a plurality of types of sequences and said probes have mutually overlapped sequences.

15. A nucleic acid information detection method according to any one of claim 1 through claim 14, wherein said probes having a plurality of types of sequences comprise overlapping probes of; a perfect matched probe having a perfect complementary sequence at least partially with said target nucleic acid sequence, one or more types of imperfect matched probes having at least one partial mutation in said perfect matched probe, and said perfect matched probe and said imperfect matched probe having an extended or shortened base sequence on both ends or one end.

16. A nucleic acid information detection method according to any one of claim 1 through claim 15, further comprising a step of comparing an analysis result of a probe group having a lower Tm value among the overlapping probes with an analysis result of a probe group having a higher Tm value, thereby deciding the nucleic acid information.

17. A nucleic acid information detection method according to any one of claim 1 through claim 16, wherein the probes have sequences (SEQ ID NO: 59 to 69) comprising 20mer base sequences for analyzing K-ras codon12.

18. A nucleic acid information detection method according to any one of claim 1 through claim 17,wherein the probes have sequences (SEQ ID NO: 70 to 83) comprising 17mer base sequences for analyzing K-ras codon12.

19. A nucleic acid information detection method according to any one of claim 1 through claim 18,wherein the probes consist of probes having sequences of (SEQ ID NO: 56 to 83) 17mer base sequences for analyzing K-ras codon12, and probes having sequences of (SEQ ID NO: 70 to 83) 20mer base sequences for analyzing K-ras codon12.

20. A nucleic acid information detection method according to any one of claim 1 through claim 19,wherein said hybrid formation is performed by making a liquid sample including a target nucleic acid contact with a probe fixed onto a porous body.

21. A nucleic acid information detection method according to claim 20, further comprising a step of making said liquid sample reciprocate once or a plurality of times in said porous body.

22. A nucleic acid information detection method according to any one of claim 1 through claim 21, wherein said signal is detected based on detection of a fluorescent marker.

23. A nucleic acid information detection method according to any one of claim 1 through claim 22, wherein said target nucleic acid is any one of an oncogene, an intracellular drug resistance gene, a cell cycle regulator gene, and an apoptosis related gene, or a combination of these.

24. A nucleic acid information detection apparatus comprising: a sample storage container for containing a sample including a target nucleic acid; a nucleic acid reaction carrier including a porous structure which can fix said nucleic acid and connected to said container; a driving device for mobilizing said sample under control, between said container and said nucleic acid reaction carrier without leaking; a temperature control device for controlling a reaction temperature on said reaction carrier; and a device for detecting a signal from a hybrid between a target nucleic acid and probes formed on said porous structure.

25. A nucleic acid information detection apparatus according to claim 24, further comprising: one or more solution storage containers for storing solutions connected to said nucleic acid reaction carrier and to contain types of solutions different to the sample solution including the target nucleic acid; and a device which appropriately mixes the various solutions contained in said solution storage containers and sends these to said nucleic acid reaction carrier.

26. A nucleic acid information detection apparatus according to either one of claim 24 and claim 25, wherein said target nucleic acid is any one of an oncogene, an intracellular drug resistance gene, a cell cycle regulator gene, and a apoptosis related gene, or a combination of these.

## Amended claims

### Amended claims under Art. 19.1 PCT

**1.** (amended). A nucleic acid information detection method wherein a target nucleic acid and probes made solid phase on a carrier and having a complementary sequence with at least a portion of said target nucleic acid sequence are contacted with each other in order to form hybrids between said target nucleic acid and said probes, and the amount of signal generated depending on the amount of hybrids is measured in order to detect the information on the target nucleic acid,
said method including kinetically obtaining data of said signal.

**2.** (as is). A nucleic acid information detection method according to claim 1, wherein obtaining the data of said signal is performed while changing a measurement condition or a detection condition of a reaction.

**3.** (as is). A nucleic acid information detection method according to claim 2, wherein obtaining the data of said signai is performed while changing at least one of; a reaction temperature, a composition, a volume, and a type of reaction solution.

**4.** (as is). A nucleic acid information detection method according to claim 3, wherein said change is for the reaction temperature.

**5.** (as is). A nucleic acid information detection method wherein a perfect matched probe having a perfect complementary sequence with respect to at least part of a target nucleic acid sequence, and one or more types of imperfectly matched probes having at least one part of the perfect matched probe mutated are contacted with said target nucleic acid in order to form hybrids between said target nucleic acid, and said perfect matched probe or said imperfect matched probes, so that the information on the target nucleic acid can be detected based on a difference in binding strength of the hybrids,
said method including kinetically obtaining data of said signal while changing continuously or stepwise the condition for measuring or detecting the signal from said hybrids.

**6.** (as is). A nucleic acid information detection method according to claim 5, wherein obtaining the data of said signal is performed while changing at least one of; a reaction temperature, a composition, a volume, and a type of reaction solution.

**7.** (as is). A nucleic acid information detection method according to claim 6, wherein said change is for the reaction temperature

**8.** (as is). A nucleic acid information detection method according to claim 7, wherein said change of the reaction temperature is to increase the temperature from a temperature lower than a Tm value of the hybrids to be detected to a temperature higher than the Tm value.

**9.** (as is). A nucleic acid information detection method according to claim 7, wherein said change of the reaction temperature is a temperature cycle of one or more times comprising increase and decrease between a temperature lower than the Tm value to a temperature higher than the Tm value.

**10.** (as is). A nucleic acid information detection method according to either claim 8 or claim 9, including a step of measuring a maximum value of the signal strength while increasing said temperature.

**11.** (as is). A nucleic acid information detection method according to either claim 8 or claim 9, including a step of measuring an amount of change in the signal strength while increasing said temperature.

**12.** (as is). A nucleic acid information detection method according to any one of claim 5 through claim 11, further comprising the steps of continuously or stepwise increasing the temperature at which a signal from said hybrid is measured, measuring the change in the signal strength from said hybrid between respective temperatures, and maintaining the temperature when the amount of change starts to decrease.

**13.** (as is). A nucleic acid information detection method according to any one of claim 1 through claim 12, wherein in an identical system where identical reaction conditions are applicable, a plurality of types of probes are used in order to detect the information on a plurality of types of nucleic acids at the same time.

**14.** (as is). A nucleic acid information detection method according to any one of claim 1 through claim 13, wherein said probes are a plurality of types of probes having a plurality of types of sequences and said probes have mutually overlapped sequences.

**15.** (as is). A nucleic acid information detection method according to any one of claim 1 through claim 14, wherein said probes having a plurality of types of sequences comprise overlapping probes of; a perfect matched probe having a perfect complementary sequence at least partially with said target nucleic acid sequence, one or more types of imperfect matched probes having at least one partial mutation in said perfect matched probe, and said perfect matched probe and said imperfect matched probe having an extended or shortened base sequence on both ends or one end.

**16.** (as is). A nucleic acid information detection method according to any one of claim 1 through claim 15, further comprising a step of comparing an analysis result of a probe group having a lower Tm value among the overlapping probes with an analysis result of a probe group having a higher Tm value, thereby deciding the nucleic acid information.

**17.** (as is). A nucleic acid information detection method according to any one of claim 1 through claim 16, wherein the probes have sequences (SEQ ID NO: 59 to 69) comprising 20mer base sequences for analyzing K-ras codon12.

**18.** (as is). A nucleic acid information detection method according to any one of claim 1 through claim 17, wherein the probes have sequences (SEQ ID NO: 70 to 83) comprising 17mer base sequences for analyzing K-ras codon12.

**19.** (as is). A nucleic acid information detection method according to any one of claim 1 through claim 18, wherein the probes consist of probes having sequences of (SEQ ID NO: 56 to 83) decahaptamer base sequences for analyzing K-ras codon12, and probes having sequences of (SEQ ID NO: 70 to 83) 20mer base sequences for analyzing K-ras codon12.

**20.** (as is). A nucleic acid information detection method according to any one of claim 1 through claim 19, wherein said hybrid formation is performed by making a liquid sample including a target nucleic acid contact with a probe fixed onto a porous body.

**21.** (as is). A nucleic acid information detection method according to claim 20, further comprising a step of making said liquid sample reciprocate once or a plurality of times in said porous body.

**22.** (as is). A nucleic acid information detection method according to any one of claim 1 through claim 21, wherein said signal is detected based on detection of a fluorescent marker.

**23.** (as is). A nucleic acid information detection method according to any one of claim 1 through claim 22, wherein said target nucleic acid is any one of an oncogene, an intracellular drug resistance gene, a cell cycle regulator gene, and an apoptosis related gene, or a combination of these.

**24.** (as is). A nucleic acid information detection apparatus comprising: a sample storage container for containing a sample including a target nucleic acid; a nucleic acid reaction carrier including a porous structure which can fix said nucleic acid and connected to said container; a driving device for mobilizing said sample under control, between said container and said nucleic acid reaction carrier without leaking; a temperature control device for controlling a reaction temperature on said reaction carrier; and a device for detecting a signal from a hybrid between a target nucleic acid and probes formed on said porous structure.

**25.** (as is). A nucleic acid information detection apparatus according to claim 24, further comprising: one or more solution storage containers for storing solutions connected to said nucleic acid reaction carrier and to contain types of solutions different to the sample solution including the target nucleic acid; and a device which appropriately mixes the various solutions contained in said solution storage containers and sends these to said nucleic acid reaction carrier.

**26.** (as is). A nucleic acid information detection apparatus according to either one of claim 24 and claim 25, wherein said target nucleic acid is any one of an oncogene, an intracellular drug resistance gene, a cell cycle regulator gene, and a apoptosis related gene, or a combination of these.


SEQUENCE LISTING

<110> Olympus Optical Co., Ltd.
<120> A Method of Detecting Nucleic Acid Information and Device Therefor
<130> PC8810
<160> 83

<210> SEQ ID NO:1
<211> 18
<212> DNA
<213> Artificial Sequence
<400> 1
atcatctaga cagagatc 18

<210> SEQ ID NO:2
<211> 18
<212> DNA
<213>
<400> 2
atcatctaga gagagatc 18

<210> SEQ ID NO:3
<211> 18
<212> DNA
<213> Artificial Sequence
<400> 3
atcatctaga aagagatc 18

<210> SEQ ID NO:4
<211> 18
<212> DNA
<213> Artificial Sequence
<400> 4
atcatctaga tagagatc 18

<210> SEQ ID NO:5
<211> 18
<212> DNA

<213> Artificial Sequence
<400> 5
gatctctgtc tagatgat 18

<210> SEQ ID NO:6
<211> 98
<212> DNA
<213> Homo sapiens
<400> 6
tggctctgac tgtaccacca tccactacaa ctacatatgt aacagttcct gcatgggcgg 60 catgaaccgg aggcccatcc
tcaccatcat cacactgg 98

<210> SEQ ID NO:7
<211> 21
<212> DNA
<213> Homo sapiens
<400> 7
acaactacat atgtaacagt t 21

<210> SEQ ID NO:8
<211> 21
<212> DNA
<213> Homo sapiens
<400> 8
aactgttaca catgtagttg t 21

<210> SEQ ID NO:9
<211> 21
<212> DNA
<213> artificial
<400> 9
aactgttaca gatgtagttg t 21

<210> SEQ ID NO:10
<211> 21
<212> DNA
<213> Homo sapiens
<400> 10

aactgttaca tatgtagttg t 21

<210> SEQ ID NO:11
<211> 21
<212> DNA
<213> artificial
<400> 11
aactgttaca aatgtagttg t 21

<210> SEQ ID NO:12
<211> 20
<212> DNA
<213> Homo sapiens
<400> 12
gactgaatat aaacttgtgg 20

<210> SEQ ID NO:13
<211> 20
<212> DNA
<213> Homo sapiens
<400> 13
ctattgttgg atcatattcg 20

<210> SEQ ID NO:14
<211> 20
<212> DNA
<213> artificial
<400> 14
cctacgccac cagctccaac 20

<210> SEQ ID NO:15
<211> 20
<212> DNA
<213> Homo sapiens
<400> 15
gttggagctg ttggcgtagg 20

<210> SEQ ID NO:16

<211> 20
<212> DNA
<213> Homo sapiens
<400> 16
gttggagctg atggcgtagg 20

<210> SEQ ID NO:17
<211> 20
<212> DNA
<213> Homo sapiens
<400> 17
gttggagctg ctggcgtagg 20

<210> SEQ ID NO:18
<211> 20
<212> DNA
<213> Homo sapiens
<400> 18
gttggagcta gtggcgtagg 20

<210> SEQ ID NO:19
<211> 20
<212> DNA
<213> Homo sapiens
<400> 19
gttggagctt gtggcgtagg 20

<210> SEQ ID NO:20
<211> 20
<212> DNA
<213> Homo sapiens
<400> 20
gttggagctc gtggcgtagg 20

<210> SEQ ID NO:21
<211> 20
<212> DNA
<213> Homo sapiens

<400> 21
gttggagctg gtggcgtagg 20

<210> SEQ ID NO:22
<211> 20
<212> DNA
<213> Homo sapiens
<400> 22
tggctctgac tgtaccacca 20

<210> SEQ ID NO:23
<211> 20
<212> DNA
<213> Homo sapiens
<400> 23
ccagtgtgat gatggtgagg 20

<210> SEQ ID NO:24
<211> 20
<212> DNA
<213> Homo sapiens
<400> 24
gactgaatat aaacttgtgg 20

<210> SEQ ID NO:25
<211> 20
<212> DNA
<213> Homo sapiens
<400> 25
ctattgttgg atcatattcg 20

<210> SEQ ID NO:26
<211> 20
<212> DNA
<213> Homo sapiens
<400> 26
cctacgccag cagctccaac 20

<210> SEQ ID NO:27
<211> 20
<212> DNA
<213> Homo sapiens
<400> 27
cctacgccat cagctccaac 20

<210> SEQ ID NO:28
<211> 20
<212> DNA
<213> Homo sapiens
<400> 28
cctacgccaa cagctccaac 20

<210> SEQ ID NO:29
<211> 22
<212> DNA
<213> artificial
<400> 29
catgtatcga ggataaatga ag 22

<210> SEQ ID NO:30
<211> 20
<212> DNA
<213> Homo sapiens
<400> 30
cctacgccac cagctccaac 20

<210> SEQ ID NO:31
<211> 20
<212> DNA
<213> Homo sapiens
<400> 31
cctacgccac gagctccaac 20

<210> SEQ ID NO:32
<211> 20
<212> DNA

<213> Homo sapiens
<400> 32
cctacgccac aagctccaac 20

<210> SEQ ID NO:33
<211> 20
<212> DNA
<213> Homo sapiens
<400> 33
cctacgccac tagctccaac 20

<210> SEQ ID NO:34
<211> 20
<212> DNA
<213> Homo sapiens
<400> 34
gttggagctg ctggcgtagg 20

<210> SEQ ID NO:35
<211> 20
<212> DNA
<213> Homo sapiens
<400> 35
gttggagctg atggcgtagg 20

<210> SEQ ID NO:36
<211> 20
<212> DNA
<213> Homo sapiens
<400> 36
gttggagctg ttggcgtagg 20

<210> SEQ ID NO:37
<211> 20
<212> DNA
<213> Homo sapiens
<400> 37
gttggagctg gtggcgtagg 20

<210> SEQ ID NO:38
<211> 20
<212> DNA
<213> Homo sapiens
<400> 38
gttggagctc gtggcgtagg 20

<210> SEQ ID NO:39
<211> 20
<212> DNA
<213> Homo sapiens
<400> 39
gttggagctt gtggcgtagg 20

<210> SEQ ID NO:40
<211> 20
<212> DNA
<213> Homo sapiens
<400> 40
gttggagcta gtggcgtagg 20

<210> SEQ ID NO:41
<211> 21
<212> DNA
<213> Homo sapiens
<400> 41
aactgttaca catgtagttg t 21

<210> SEQ ID NO:42
<211> 21
<212> DNA
<213> Homo sapiens
<400> 42
aactgttaca gatgtagttg t 21

<210> SEQ ID NO:43
<211> 21

<212> DNA
<213> Homo sapiens
<400> 43
aactgttaca tatgtagttg t 21

<210> SEQ ID NO:44
<211> 21
<212> DNA
<213> Homo sapiens
<400> 44
aactgttaca aatgtagttg t 21

<210> SEQ ID NO:45
<211> 21
<212> DNA
<213> Homo sapiens
<400> 45
acaactacat gtgtaacagt t 21

<210> SEQ ID NO:46
<211> 21
<212> DNA
<213> Homo sapiens
<400> 46
acaactacat ctgtaacagt t 21

<210> SEQ ID NO:47
<211> 21
<212> DNA
<213> Homo sapiens
<400> 47
acaactacat atgtaacagt t 21

<210> SEQ ID NO:48
<211> 21
<212> DNA
<213> Homo sapiens
<400> 48

acaactacat ttgtaacagt t 21

<210> SEQ ID NO:49
<211> 21
<212> DNA
<213> Homo sapiens
<400> 49
acaactacag atgtaacagt t 21

<210> SEQ ID NO:50
<211> 21
<212> DNA
<213> Homo sapiens
<400> 50
acaactacat atgtagcagt t 21

<210> SEQ ID NO:51
<211> 21
<212> DNA
<213> Homo sapiens
<400> 51
acaagtacat atgtaacagt t 21

<210> SEQ ID NO:52
<211> 21
<212> DNA
<213> Homo sapiens
<400> 52
acaagtacat atgtagcagt t 21

<210> SEQ ID NO:53
<211> 21
<212> DNA
<213> Homo sapiens
<400> 53
acaagtacag acgtagcagt t 21

<210> SEQ ID NO:54

<211> 21
<212> DNA
<213> Homo sapiens
<400> 54
acaactacat gtgtaacagt t 21

<210> SEQ ID NO:55
<211> 20
<212> DNA
<213> Homo sapiens
<400> 55
ggtggagctg gtggcgtagg 20

<210> SEQ ID NO:56
<211> 20
<212> DNA
<213> homo sapiens
<400> 56
gttggagctg gtggcgtagg 20

<210> SEQ ID NO:57
<211> 20
<212> DNA
<213> homo sapiens
<400> 57
gttggagctc gtggcgtagg 20

<210> SEQ ID NO:58
<211> 20
<212> DNA
<213> homo sapiens
<400> 58
gttggagctt gtggcgtagg 20

<210> SEQ ID NO:59
<211> 20
<212> DNA
<213> homo sapiens

<400> 59
gttggagcta gtggcgtagg 20

<210> SEQ ID NO:60
<211> 20
<212> DNA
<213> homo sapiens
<400> 60
gttggagctg ctggcgtagg 20

<210> SEQ ID NO:61
<211> 20
<212> DNA
<213> homo sapiens
<400> 61
gttggagctg atggcgtagg 20

<210> SEQ ID NO:62
<211> 20
<212> DNA
<213> homo sapiens
<400> 62
gttggagctg ttggcgtagg 20

<210> SEQ ID NO:63
<211> 20
<212> DNA
<213> homo sapiens
<400> 63
cctacgccac cagctccaac 20

<210> SEQ ID NO:64
<211> 20
<212> DNA
<213> homo sapiens
<400> 64
cctacgccac gagctccaac 20

<210> SEQ ID NO:65
<211> 20
<212> DNA
<213> homo sapiens
<400> 65
cctacgccac aagctccaac 20

<210> SEQ ID NO:66
<211> 20
<212> DNA
<213> homo sapiens
<400> 66
1cctacgccac tagctccaac 20

<210> SEQ ID NO:67
<211> 20
<212> DNA
<213> homo sapiens
<400> 67
cctacgccag cagctccaac 20

<210> SEQ ID NO:68
<211> 20
<212> DNA
<213> homo sapiens
<400> 68
cctacgccat cagctccaac 20

<210> SEQ ID NO:69
<211> 20
<212> DNA
<213> homo sapiens
<400> 69
cctacgccaa cagctccaac 20

<210> SEQ ID NO:70
<211> 17
<212> DNA

<213> homo sapiens
<400> 70
ttggagctgg tggcgta 17

<210> SEQ ID NO:71
<211> 17
<212> DNA
<213> homo sapiens
<400> 71
ttggagctcg tggcgta 17

<210> SEQ ID NO:72
<211> 17
<212> DNA
<213> homo sapiens
<400> 72
ttggagcttg tggcgta 17

<210> SEQ ID NO:73
<211> 17
<212> DNA
<213> homo sapiens
<400> 73
ttggagctag tggcgta 17

<210> SEQ ID NO:74
<211> 17
<212> DNA
<213> homo sapiens
<400> 74
ttggagctgc tggcgta 17

<210> SEQ ID NO:75
<211> 17
<212> DNA
<213> homo sapiens
<400> 75
ttggagctga tggcgta 17

<210> SEQ ID NO:76
<211> 17
<212> DNA
<213> homo sapiens
<400> 76
ttggagctgt tggcgta 17

<210> SEQ ID NO:77
<211> 17
<212> DNA
<213> homo sapiens
<400> 77
tacgccacca gctccaa 17

<210> SEQ ID NO:78
<211> 17
<212> DNA
<213> homo sapiens
<400> 78
tacgccacga gctccaa 17

<210> SEQ ID NO:79
<211> 17
<212> DNA
<213> homo sapiens
<400> 79
tacgccacaa gctccaa 17

<210> SEQ ID NO:80
<211> 17
<212> DNA
<213> homo sapiens
<400> 80
tacgccacta gctccaa 17

<210> SEQ ID NO:81
<211> 17

<212> DNA
<213> homo sapiens
<400> 81
tacgccagca gctccaa 17

<210> SEQ ID NO:82
<211> 17
<212> DNA
<213> homo sapiens
<400> 82
tacgccatca gctccaa 17

<210> SEQ ID NO:83
<211> 17
<212> DNA
<213> homo sapiens
<400> 83
tacgccaaca gctccaa 17